# EUROPEAN PATENT APPLICATION

(11) **EP 4 793 347 A1**
(43) Date of publication of application: **19.08.2026**
(21) Application number: 25157462.0
(22) Date of filing: 12.02.2025
(51) Int. Cl.: C12N 9/10, C12N 9/16, C12P 7/6409

(54) **VARIANTS OF METAZOAN FATTY ACID SYNTHASE FOR THE PRODUCTION OF FATTY ACIDS, ALDEHYDES, AND ALCOHOLS**

(71) Applicant: Johann-Wolfgang-Goethe-Universität Frankfurt am Main, 60323 Frankfurt am Main (DE)
(72) Inventor: Grininger, Martin, 60438 Frankfurt (DE); Ludig, Damian, 60438 Frankfurt (DE); Rittner, Alexander, 60438 Frankfurt (DE); Gusenda, Christian, 60438 Frankfurt (DE)
(74) Representative: Engelhard, Markus

(57) **Abstract**

The present invention relates to a protein comprising one or more polypeptide chains, wherein said one or more polypeptide chains comprise (i) an amino acid sequence encoding a metazoan ketoacyl synthase domain and/or (ii) an amino acid sequence encoding a bacterial thioesterase domain or an amino acid sequence encoding a thioreductase domain. The present invention further relates to a nucleic acid molecule encoding the protein and to a host cell comprising the protein and/or the nucleic acid molecule. Furthermore, the present invention relates to a method for producing a fatty acid, a fatty alcohol, and/or a fatty aldehyde, and to a use of the protein, the nucleic acid molecule, or the host cell for producing a fatty acid, a fatty alcohol, and/or a fatty aldehyde.

## Description

### FIELD OF THE INVENTION

The present invention relates to a protein comprising one or more polypeptide chains, wherein said one or more polypeptide chains comprise (i) an amino acid sequence encoding a metazoan ketoacyl synthase domain and/or (ii) an amino acid sequence encoding a bacterial thioesterase domain or an amino acid sequence encoding a thioreductase domain. The present invention further relates to a nucleic acid molecule encoding the protein and to a host cell comprising the protein and/or the nucleic acid molecule. Furthermore, the present invention relates to a method for producing a fatty acid, a fatty alcohol, and/or a fatty aldehyde, and to a use of the protein, the nucleic acid molecule, or the host cell for producing a fatty acid, a fatty alcohol, and/or a fatty aldehyde.

### BACKGROUND OF THE INVENTION

The sustainable production of hydrocarbons through biocatalytic pathways presents significant opportunities. The precise biosynthesis of fatty acid (FA) of specific chain length is of high interest for chemical industry. For example, short chain fatty acids (SCFAs), particularly fatty acids with ≤C₈, have extensive applications in the food, pharmaceuticals and cosmetics, and have recently been shown to have potential in the prevention and treatment of immune-mediated diseases, while medium chain fatty acids (MCFAs), particularly fatty acids with C₁₀-C₁₄, can be directly used as lubricants, fragrances, paint additives, and pharmaceuticals. The biotechnological production of SCFAs and MCFAs offers a potential sustainable alternative to land-dependent coconut and palm oil extraction.

Approaches for the bioproduction of SCFAs and MCFAs have been based on the eukaryotic multienzyme FASs and the bacterial FAS system composed of separate enzymes. However, beyond titers lagging behind industrial demands, approaches have suffered so far from the lack of specificity in the production of the desired chain length.

Thus, there is the need for means that enable an efficient production of FAs, particularly FAs with specific chain lengths. Furthermore, there is a need for improved means for producing fatty acids in microorganisms, in particular for producing SCFAs and MCFAs. There is also the need for means that allow a precise control of hydrocarbon chain length. Moreover, there is the need for means that allow to produce fatty acids, fatty alcohols, and/or fatty aldehydes with a specific chain length.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a protein, preferably a fatty acid synthase, comprising one or more polypeptide chains, wherein said one or more polypeptide chains comprise
(i) an amino acid sequence encoding a metazoan ketoacyl synthase (KS) domain, preferably an amino acid sequence encoding a murine KS domain; and/or
(ii) an amino acid sequence encoding a bacterial thioesterase ('TesA) domain, preferably an amino acid sequence encoding a thioesterase domain of E. coli; or
   an amino acid sequence encoding a thioreductase (TR) domain, preferably an amino acid sequence encoding a bacterial TR domain.

In one embodiment, said amino acid sequence encoding a metazoan KS domain has at least 70%, preferably at least 80%, more preferably at least 90%, even more preferably at least 95% sequence identity to the amino acid sequence of SEQ ID NO: 1;
said amino acid sequence encoding a bacterial 'TesA domain has at least 70%, preferably at least 80%, more preferably at least 90%, even more preferably at least 95% sequence identity to the amino acid sequence of SEQ ID NO: 2; and/or
said amino acid sequence encoding a TR domain has at least 70%, preferably at least 80%, more preferably at least 90%, even more preferably at least 95% sequence identity to the amino acid sequence of SEQ ID NO: 3, 4, or 5.

In one embodiment, the amino acid sequence encoding a metazoan KS domain has at least 70%, preferably at least 80%, more preferably at least 90%, even more preferably at least 95% sequence identity to the amino acid sequence of SEQ ID NO: 1 and comprises an amino acid substitution in the KS domain at a position corresponding to G113, S117, or T196 of the amino acid sequence of SEQ ID NO: 1;
wherein, preferably, said amino acid substitution is selected from G113S, G113M, G113F, G113W, G113L, G113I, G113T, G113H, G113Y, S117R, S117Y, and T196L;
wherein, more preferably, said amino acid substitution is selected from G113S, G113M, G113F, G113W, G113L, G113T, G113H, and G113Y;
wherein, even more preferably, said amino acid substitution is selected from G113S, G113M, G113F, and G113W.

In one embodiment, the amino acid sequence encoding a 'TesA domain has at least 70%, preferably at least 80%, more preferably at least 90%, even more preferably at least 95% sequence identity to the amino acid sequence of SEQ ID NO: 2 and comprises one or more amino acid substitutions in the 'TesA domain at a position corresponding to L109, M141, E142, Y145, and/or L146 of the amino acid sequence of SEQ ID NO: 2;
wherein, preferably, said one or more amino acid substitutions are selected from L109P, M141L, E142D, Y145G, L146K, and combinations thereof;
wherein, more preferably, the amino acid sequence encoding a 'TesA domain comprises amino acid substitution L109P or comprises amino acid substitutions M141L, E142D, Y145G, and L146K.

In one embodiment, the TR domain is a TR domain of a *Mycobacterium* sp., a *Nocardia* sp., *a Tsukamurella* sp., a *Neurospora* sp., a *Stigmatella* sp., or *Methanobrevibacter* sp.; optionally a TR domain of a carboxylic acid reductase (CAR) or of a hybrid polyketide synthase non-ribosomal peptide synthetase (PKS-NRPS), preferably a TR domain of a CAR; wherein, preferably, the TR domain is a TR domain of *Mycobacterium marinum, Mycobacterium phlei, Mycobacterium smegmatis, Mycobacterium tuberculosis, Nocardia iowensis, Nocardia otitidiscaviarum, Tsukamurella paurometabola, Neurospora crassa, Stigmatella aurantiaca,* or *Methanobrevibacter ruminantium;*
wherein, more preferably, the TR domain is a TR domain of *Mycobacterium phlei, Mycobacterium smegmatis,* or *Nocardia otitidiscaviarum,* optionally a TR domain of a CAR of *Mycobacterium phlei, Mycobacterium smegmatis,* or *Nocardia otitidiscaviarum.*

In one embodiment, the amino acid sequence encoding a TR domain has at least 70%, preferably at least 80%, more preferably at least 90%, even more preferably at least 95% sequence identity to the amino acid sequence of SEQ ID NO: 3, 4, or 5.

In one embodiment, said one or more polypeptide chains comprise
(i) said amino acid sequence encoding a metazoan ketoacyl synthase (KS) domain, preferably said amino acid sequence encoding a murine KS domain; and
(ii) said amino acid sequence encoding a bacterial thioesterase ('TesA) domain, preferably said amino acid sequence encoding a thioesterase domain of E. coli; or said amino acid sequence encoding a thioreductase (TR) domain, preferably said amino acid sequence encoding a bacterial TR domain;
or said one or more polypeptide chains comprise
(i) said amino acid sequence encoding a metazoan ketoacyl synthase (KS) domain; wherein the amino acid sequence encoding a metazoan KS domain has at least 70%, preferably at least 80%, more preferably at least 90%, even more preferably at least 95% sequence identity to the amino acid sequence of SEQ ID NO: 1 and comprises an amino acid substitution in the KS domain at a position corresponding to G113, S117, or T196 of the amino acid sequence of SEQ ID NO: 1;
   wherein, preferably, said amino acid substitution is selected from G113S, G113M, G113F, G113W, G113L, G113I, G113T, G113H, G113Y, S117R, S117Y, and T196L;
   wherein, more preferably, said amino acid substitution is selected from G113S, G113M, G113F, G113W, G113L, G113T, G113H, and G113Y; wherein, even more preferably, said amino acid substitution is selected from G113S, G113M, G113F, and G113W; and
(ii) an amino acid sequence encoding a thioesterase domain, preferably a metazoan thioesterase domain, more preferably a thioesterase domain having at least 70%, preferably at least 80%, more preferably at least 90%, even more preferably at least 95% sequence identity to the amino acid sequence of SEQ ID NO: 35.

In one embodiment, the one or more polypeptide chains comprise
- an amino acid sequence encoding a metazoan KS domain having at least 70%, preferably at least 80%, more preferably at least 90%, even more preferably at least 95% sequence identity to the amino acid sequence of SEQ ID NO: 1; and
   an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1; and
   an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity to the amino acid sequence of SEQ ID NO: 1; and
   an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113S; and
   an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113S; and
   an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113S; and
   an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113M; and
   an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113M; and
   an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113M; and
   an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113F; and
   an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113F; and
   an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113F; and
   an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113W; and
   an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113W; and
   an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113W; and
   an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113T; and
   an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113L; and
   an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113H; and
   an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113Y; and
   an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113S; and
   an amino acid sequence encoding a TR domain having at least 70%, preferably at least 80%, more preferably at least 90%, even more preferably at least 95% sequence identity to the amino acid sequence of SEQ ID NO: 3, 4, or 5; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113M; and
   an amino acid sequence encoding a TR domain having at least 70%, preferably at least 80%, more preferably at least 90%, even more preferably at least 95% sequence identity to the amino acid sequence of SEQ ID NO: 3, 4, or 5; or
- an amino acid sequence encoding a metazoan KS domain having at least 70%, preferably at least 80%, more preferably at least 90%, even more preferably at least 95% sequence
   identity to the amino acid sequence of SEQ ID NO: 1 and comprising the amino acid substitution G113F; and
   an amino acid sequence encoding a TR domain having at least 70%, preferably at least 80%, more preferably at least 90%, even more preferably at least 95% sequence identity
   to the amino acid sequence of SEQ ID NO: 3, 4, or 5; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113W; and
   an amino acid sequence encoding a TR domain having at least 70%, preferably at least 80%, more preferably at least 90%, even more preferably at least 95% sequence identity to the amino acid sequence of SEQ ID NO: 3, 4, or 5.

In one embodiment, said protein comprises an amino acid sequence selected from
SEQ ID NO: 6 [mFASWT-'TesA],
SEQ ID NO: 7 [mFASWT-'TesAL109P],
SEQ ID NO: 8 [mFASWT-'TesA4x],
SEQ ID NO: 9 [mFASG113S-TE_WT],
SEQ ID NO: 10 [mFASG113S-'TesA],
SEQ ID NO: 11 [mFASG113S-"TesAL109P],
SEQ ID NO: 12 [mFASG113S-'TesA4x],
SEQ ID NO: 13 [mFASG113M-TE_WT],
SEQ ID NO: 14 [mFASG113M-'TesA],
SEQ ID NO: 15 [mFASG113M-'TesAL109P],
SEQ ID NO: 16 [mFASG113M-'TesA4x],
SEQ ID NO: 17 [mFASG113F-TE_WT],
SEQ ID NO: 18 [mFASG113F-'TesA],
SEQ ID NO: 19 [mFASG113F-'TesAL109P],
SEQ ID NO: 20 [mFASG113F-'TesA4x],
SEQ ID NO: 21 [mFASG113W-TE_WT],
SEQ ID NO: 22 [mFASG113W-'TesA],
SEQ ID NO: 23 [mFASG113W-'TesAL109P],
SEQ ID NO: 24 [mFASG113W-'TesA4x],
SEQ ID NO: 31 [mFASG113T-'TesA],
SEQ ID NO: 32 [mFASG113L-'TesA],
SEQ ID NO: 33 [mFASG113H-'TesA], and
SEQ ID NO: 34 [mFASG113Y-'TesA];
wherein, preferably, said protein comprises an amino acid sequence selected from
SEQ ID NO: 10 [mFASG113S-'TesA],
SEQ ID NO: 11 [mFASG113S-"TesAL109P],
SEQ ID NO: 12 [mFASG113S-'TesA4x],
SEQ ID NO: 13 [mFASG113M-TE_WT],
SEQ ID NO: 14 [mFASG113M-'TesA],
SEQ ID NO: 15 [mFASG113M-'TesAL109P],
SEQ ID NO: 16 [mFASG113M-'TesA4x],
SEQ ID NO: 18 [mFASG113F-'TesA],
SEQ ID NO: 19 [mFASG113F-'TesAL109P],
SEQ ID NO: 20 [mFASG113F-'TesA4x],
SEQ ID NO: 22 [mFASG113W-'TesA],
SEQ ID NO: 23 [mFASG113W-'TesAL109P],
SEQ ID NO: 24 [mFASG113W-'TesA4x],
SEQ ID NO: 31 [mFASG113T-'TesA],
SEQ ID NO: 32 [mFASG113L-'TesA],
SEQ ID NO: 33 [mFASG113H-'TesA], and
SEQ ID NO: 34 [mFASG113Y-'TesA];
wherein, optionally, said protein comprises an amino acid sequence selected from
SEQ ID NO: 10 [mFASG113S-'TesA],
SEQ ID NO: 12 [mFASG113S-'TesA4x],
SEQ ID NO: 14 [mFASG113M-'TesA], and
SEQ ID NO: 16 [mFASG113M-'TesA4x],
such as an amino acid sequence selected from
SEQ ID NO: 12 [mFASG113S-'TesA4x] and
SEQ ID NO: 14 [mFASG113M-'TesA].

In one embodiment, said protein comprises an amino acid sequence selected from
SEQ ID NO: 25 [mFAS-TR2],
SEQ ID NO: 26 [mFAS-TR1],
SEQ ID NO: 27 [mFAS-TR3],
SEQ ID NO: 28 [mFASG113W-TR2],
SEQ ID NO: 29 [mFASG113W-TR1], and
SEQ ID NO: 30 [mFASG113W-TR3];
wherein, preferably, said protein comprises an amino acid sequence selected from
SEQ ID NO: 25 [mFAS-TR2]
SEQ ID NO: 28 [mFASG113W-TR2],
SEQ ID NO: 29 [mFASG113W-TR1], and
SEQ ID NO: 30 [mFASG113W-TR3];
wherein, more preferably, said protein comprises an amino acid sequence selected from
SEQ ID NO: 28 [mFASG113W-TR2],
SEQ ID NO: 29 [mFASG113W-TR1], and
SEQ ID NO: 30 [mFASG113W-TR3];
wherein, even more preferably, said protein comprises an amino acid sequence of SEQ ID NO: 28 [mFASG113W-TR2].

In one embodiment, the protein does not comprise a thioesterase (TE) domain.

In a further aspect, the present invention relates to a nucleic acid molecule encoding a protein, said protein being as defined herein.

In a further aspect, the present invention relates to a host cell, comprising a protein as defined herein, and/or comprising a nucleic acid molecule as defined herein;
wherein, optionally, said host cell is a yeast cell.

In a further aspect, the present invention relates to a method for producing a fatty acid, a fatty alcohol, and/or a fatty aldehyde, comprising contacting the protein, as defined herein, with a substrate selected from acetyl-CoA, malonyl-CoA, NADPH, and combinations thereof; and/or
comprising expressing a nucleic acid molecule encoding a protein, said nucleic acid molecule being a nucleic acid molecule as defined herein, in a cell, preferably in a host cell as defined herein.

In one embodiment, said method is a method for producing a C₈ fatty acid, wherein said protein comprises an amino acid sequence selected from
SEQ ID NO: 18 [mFASG113F-'TesA],
SEQ ID NO: 20 [mFASG113F-'TesA4x],
SEQ ID NO: 22 [mFASG113W-'TesA],
SEQ ID NO: 24 [mFASG113W-'TesA4x],
SEQ ID NO: 33 [mFASG113H-'TesA], and
SEQ ID NO: 34 [mFASG113Y-'TesA];

said method is a method for producing a C₁₀ fatty acid, wherein said protein comprises an amino acid sequence selected from
   SEQ ID NO: 10 [mFASG113S-'TesA],
   SEQ ID NO: 17 [mFASG113F-TE_WT],
   SEQ ID NO: 21 [mFASG113W-TE_WT],
   SEQ ID NO: 22 [mFASG113W-'TesA],
   SEQ ID NO: 8 [mFASWT-'TesA4x],
   SEQ ID NO: 12 [mFASG113S-'TesA4x],
   SEQ ID NO: 14 [mFASG113M-'TesA],
   SEQ ID NO: 16 [mFASG113M-'TesA4x],
   SEQ ID NO: 18 [mFASG113F-'TesA],
   SEQ ID NO: 19 [mFASG113F-'TesAL109P],
   SEQ ID NO: 20 [mFASG113F-'TesA4x],
   SEQ ID NO: 31 [mFASG113T-'TesA],
   SEQ ID NO: 32 [mFASG113L-'TesA],
   SEQ ID NO: 33 [mFASG113H-'TesA], and
   SEQ ID NO: 34 [mFASG113Y-'TesA],
   preferably an amino acid sequence of SEQ ID NO: 18 [mFASG113F-'TesA];
said method is a method for producing a C₁₂ fatty acid, wherein said protein comprises an amino acid sequence selected from
   SEQ ID NO: 17 [mFASG113F-TE_WT],
   SEQ ID NO: 18 [mFASG113F-'TesA],
   SEQ ID NO: 21 [mFASG113W-TE_WT],
   SEQ ID NO: 6 [mFASWT-'TesA],
   SEQ ID NO: 8 [mFASWT-'TesA4x],
   SEQ ID NO: 10 [mFASG113S-'TesA],
   SEQ ID NO: 11 [mFASG113S-"TesAL109P],
   SEQ ID NO: 12 [mFASG113S-'TesA4x],
   SEQ ID NO: 14 [mFASG113M-'TesA],
   SEQ ID NO: 15 [mFASG113M-'TesAL109P],
   SEQ ID NO: 16 [mFASG113M-'TesA4x],
   SEQ ID NO: 19 [mFASG113F-'TesAL109P],
   SEQ ID NO: 31 [mFASG113T-'TesA],
   SEQ ID NO: 32 [mFASG113L-'TesA],
   SEQ ID NO: 33 [mFASG113H-'TesA], and
   SEQ ID NO: 34 [mFASG113Y-'TesA];
said method is a method for producing a C₁₄ fatty acid, wherein said protein comprises an amino acid sequence selected from
   SEQ ID NO: 9 [mFASG113S-TE_WT],
   SEQ ID NO: 13 [mFASG113M-TE_WT],
   SEQ ID NO: 17 [mFASG113F-TE_WT],
   SEQ ID NO: 21 [mFASG113W-TE_WT],
   SEQ ID NO: 6 [mFASWT-'TesA],
   SEQ ID NO: 8 [mFASWT-'TesA4x],
   SEQ ID NO: 10 [mFASG113S-'TesA],
   SEQ ID NO: 11 [mFASG113S-"TesAL109P],
   SEQ ID NO: 12 [mFASG113S-'TesA4x],
   SEQ ID NO: 14 [mFASG113M-'TesA],
   SEQ ID NO: 15 [mFASG113M-'TesAL109P],
   SEQ ID NO: 16 [mFASG113M-'TesA4x],
   SEQ ID NO: 19 [mFASG113F-'TesAL109P],
   SEQ ID NO: 31 [mFASG113T-'TesA],
   SEQ ID NO: 32 [mFASG113L-'TesA], and
   SEQ ID NO: 33 [mFASG113H-'TesA];
said method is a method for producing a C₁₆ fatty acid, wherein said protein comprises an amino acid sequence selected from
   SEQ ID NO: 9 [mFASG113S-TE_WT],
   SEQ ID NO: 13 [mFASG113M-TE_WT],
   SEQ ID NO: 17 [mFASG113F-TE_WT],
   SEQ ID NO: 21 [mFASG113W-TE_WT],
   SEQ ID NO: 6 [mFASWT-'TesA],
   SEQ ID NO: 8 [mFASWT-'TesA4x],
   SEQ ID NO: 11 [mFASG113S-"TesAL109P], and
   SEQ ID NO: 15 [mFASG113M-'TesAL109P];
said method is a method for producing a C₁₈ fatty acid, wherein said protein comprises an amino acid sequence selected from
   SEQ ID NO: 9 [mFASG113S-TE_WT],
   SEQ ID NO: 13 [mFASG113M-TE_WT],
   SEQ ID NO: 17 [mFASG113F-TE_WT],
   SEQ ID NO: 21 [mFASG113W-TE_WT],
   SEQ ID NO: 7 [mFASWT-'TesAL109P],
   SEQ ID NO: 11 [mFASG113S-"TesAL109P],
   SEQ ID NO: 12 [mFASG113S-'TesA4x], and
   SEQ ID NO: 15 [mFASG113M-'TesAL109P],
   preferably an amino acid sequence of SEQ ID NO: 7 [mFASWT-'TesAL109P]; or
said method is a method for producing a C₈, C₁₀, and/or C₁₂ fatty alcohol and/or C₈, C₁₀, and/or C₁₂ fatty aldehyde, wherein said protein comprises an amino acid sequence selected from
   SEQ ID NO: 28 [mFASG113W-TR2],
   SEQ ID NO: 29 [mFASG113W-TR1], and
   SEQ ID NO: 30 [mFASG113W-TR3]; or
said method is a method for producing a C₁₆, C₁₈, and/or C₂₀ aldehyde, wherein said protein comprises an amino acid sequence of SEQ ID NO: 26 [mFAS-TR1];
wherein, preferably, said method is
a method for producing a C₈ fatty acid, wherein said protein comprises an amino acid sequence selected from
   SEQ ID NO: 20 [mFASG113F-'TesA4x] and
   SEQ ID NO: 24 [mFASG113W-'TesA4x], or
a method for producing a C₈, C₁₀, and/or C₁₂ fatty alcohol and/or C₈, C₁₀, and/or C₁₂ fatty aldehyde, wherein said protein comprises an amino acid sequence selected from
   SEQ ID NO: 28 [mFASG113W-TR2],
   SEQ ID NO: 29 [mFASG113W-TR1], and
   SEQ ID NO: 30 [mFASG113W-TR3].

In this aspect, said protein, said nucleic acid, and said host cell are as defined herein.

In a further aspect, the present invention relates to a use of a protein as defined herein, a nucleic acid molecule as defined herein, or a host cell as defined herein, for producing a fatty acid, a fatty alcohol, and/or a fatty aldehyde. In this aspect, said protein, said nucleic acid, and said host cell are as defined herein.

### DETAILED DESCRIPTION

The sustainable production of hydrocarbons via biocatalytic pathways offers chances for the development of products and processes beyond petrochemicals. Short chain (≤C8) (SCFAs) and medium chain (C10-C14) fatty acids (MCFAs), alongside their reduced derivatives fatty aldehydes and fatty alcohols, are important hydrocarbon compounds. Depending on the chain length, applications include biofuels, surfactants, lubricants and plasticizers. Metazoan fatty acid synthases (mFASs) are fast carbon-carbon bond forming multienzymes responsible for cytoplasmatic C16- and C18-FA de novo biosynthesis. Here, the inventors first describe a generalist mFAS as an engineering platform in which native chain length control is compromised. In precisely redirecting the fluxes between the FA cycle and the terminating hydrolysis, the inventors then specialize mFAS variants to produce specific subsets of SCFAs and MCFAs. This approach is highly sensitive, enabling even single amino acid exchanges to transform the generalist mFAS into variants producing C8-FAs (G113W exchange), C8/C10-FAs (G113F) or C12/C14-FAs (G113M). The inventors finally engineer mFAS variants including a thioreductase domain for the reductive release of medium chain fatty aldehydes and alcohols. A yeast cell factory, containing MCFA-producing mFAS and adapted by metabolic engineering, is capable of producing MCFAs at titers of 90.8 mg/L and purity of 83 % of total free FAs in shake flask.

As shown herein, the protein, nucleic acid molecule, host cell, method, and use of the invention enable an efficient production of FAs, particularly FAs with specific chain lengths. Furthermore, as shown herein, the protein, nucleic acid molecule, host cell, method, and use of the invention are highly effective means for producing fatty acids in microorganisms, in particular for producing SCFAs and MCFAs. The protein, nucleic acid molecule, host cell, method, and use of the invention allow for a precise control of hydrocarbon chain length. Moreover, the protein, nucleic acid molecule, host cell, method, and use of the invention are means that allow to produce fatty acids, fatty alcohols, and/or fatty aldehydes with a specific chain length.

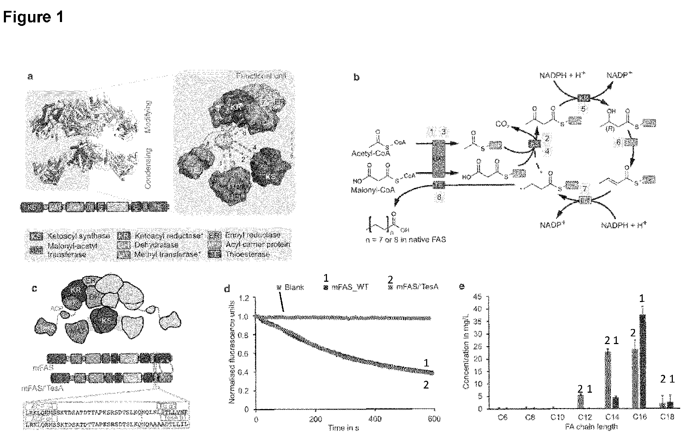

The metazoan fatty acids synthase (mFAS) forms an open 550 kDa homodimeric X-shaped fold with two reaction clefts (Figure 1a). mFAS naturally synthesizes C16- and C18-FAs through an iterative process, typically starting with acetyl-Coenzyme A (Ac-CoA) as the priming substrate and using malonyl-CoA (Mal-CoA) for elongation, with each cycle adding two carbons from malonyl to the growing fatty acyl chain. Each carbon-carbon bond formation by the ketoacyl synthase (KS) domain is followed by reduction of β-ketoacyl moiety by the β-ketoacyl reductase (KR), dehydration to enoyl by the dehydratase (DH) and further reduction by the enoyl reductase (ER) to the saturated fatty acyl. To serve this action, the enzymatic domains KS, KR, DH and ER, involved in chain extensions, are substrate-tolerant to the growing chain length of FAs. In contrast, the FA-releasing thioesterase (TE) domain of mFAS (TE^{mFAS}) is substrate specific, and very precisely intercepts synthesis after seven or eight cycles to release free C16- or C18-FAs respectively (Figure 1b). FA biosynthesis is assisted by substrate shuttling mediated by the compact four-helical bundle domain ACP, which is flexibly linked to the multienzyme FASs. The ACP covalently binds substrates and intermediates through a post-translationally introduced phosphopantetheine moiety. Their covalent attachment prevents substrates and intermediates from diffusing out of the catalytic compartment, thereby maintaining high local concentrations that enhance enzymatic efficiency.

In mFAS, the domain KS and TE regulate the length of the synthesized FAs, ensuring specificity for the desired chain lengths. The KS domain acts as the rate-limiting step in the synthetic progress, thereby determining the turnover of the FA cycle, and is crucial to the fidelity of the production of only saturated FAs. The KS hinders the production of unsaturated FA by preventing elongation of the enoyl-compounds with 42-fold reduced enzymatic efficiency compared to the correct substrate (transition state energy difference of 9 kJ/mol). Additionally, the KS domain is a key player in regulating FA chain length. The initial elongation of the acetyl moiety, priming the synthesis of one FA, proceeds with relatively little efficiency. The efficiency of KS then increases with chain length, reaching a 6.7 fold enhancement for hexanoyl moieties in the third passage of the FA cycle and a further 2.5 fold increase for decanoyl moieties in the fifth passage (corresponding to the lowering of transition state energies of 2 and 4 kJ/mol, respectively). The FA cycle slows down again when approaching the desired chain length, enabling the substrate-specific TE domain to intercept the iterative process for the release of C16- and C18-FAs.

The inventors built their approach to chain length control in mFAS on the combination of the kinetic advantages of a compartmentalized, substrate-shuttled type I biosynthesis with excellent engineerability of mFAS due to its open structural fold. The inventors first created a mFAS variant that has lost its stringent control of chain length. To this end, the inventors constructed a hybrid mFAS comprising domains for FA assembly from mFAS (KS-MAT-DH-ER-KR-ACP) and the bacterial substrate tolerant 'TesA from *E. coli* as the FA releasing TE domain. The metazoan/bacterial hybrid multienzyme turned out to operate at overall rates comparable to the native mFAS, while producing a wider range of FA chain lengths. Next, the inventors mutated the substrate binding tunnels of KS and 'TesA to alter their chain-length specificities. The inventors eventually created a focused library of 24 mFAS/'TesA hybrids combining KS and 'TesA variants that differ in the processing of short to medium acyl chains. The inventors' approach enabled the production of FAs with distinct chain lengths, overall spanning a range of C8 to C18. The inventors also created an acyl reducing hybrid with mFAS by replacing the mFAS TE with a terminating thioreductase domain (TR). After screening a set of suitable TR domains and adjusting the kinetics of the KS mediated elongation reaction, the inventors achieved direct enzymatic synthesis of alcohols and aldehydes of medium chain lengths. A yeast cell factory carrying selected mFAS/'TesA hybrids to produce C8/C10-FA or C12/C14-FA, coupled with a metabolic engineering strategy for remodeling the β-oxidation of long-chain FAs (LCFAs), enabled the production of 90.8 mg/L MCFAs (5.6 mg/L C10-FA, 74.6 mg/L C12-FA and 10.6 mg/L C14-FA), corresponding to 83 % of the detected FAs. A mFAS/TR containing strain enabled the microbial production of C10 and C12 alcohol.

As described above, the present invention relates to a protein, preferably a fatty acid synthase, comprising one or more polypeptide chains, wherein said one or more polypeptide chains comprise
(i) an amino acid sequence encoding a metazoan ketoacyl synthase (KS) domain, preferably an amino acid sequence encoding a murine KS domain; and/or
(ii) an amino acid sequence encoding a bacterial thioesterase ('TesA) domain, preferably an amino acid sequence encoding a thioesterase domain of E. coli; or
   an amino acid sequence encoding a thioreductase (TR) domain, preferably an amino acid sequence encoding a bacterial TR domain.

In one embodiment, the protein, preferably the fatty acid synthase, comprises one or more polypeptide chains, wherein said one or more polypeptide chains comprise
(i) said amino acid sequence encoding a metazoan ketoacyl synthase (KS) domain, preferably said amino acid sequence encoding a murine KS domain; and/or
(ii) said amino acid sequence encoding a bacterial thioesterase ('TesA) domain, preferably said amino acid sequence encoding a thioesterase domain of E. coli; or
   said amino acid sequence encoding a thioreductase (TR) domain, preferably said amino acid sequence encoding a bacterial TR domain;
under the proviso that, if the one or more polypeptide chains do not comprise at least one of said amino acid sequence encoding a bacterial thioesterase ('TesA) domain and said amino acid sequence encoding a thioreductase (TR) domain, then the amino acid sequence encoding a metazoan ketoacyl synthase (KS) domain has at least 70%, preferably at least 80%, more preferably at least 90%, even more preferably at least 95% sequence identity to the amino acid sequence of SEQ ID NO: 1 and comprises an amino acid substitution in the KS domain at a position corresponding to G113, S117, or T196 of the amino acid sequence of SEQ ID NO: 1; wherein, preferably, said amino acid substitution is selected from G113S, G113M, G113F, G113W, G113L, G113I, G113T, G113H, G113Y, S117R, S117Y, and T196L;
wherein, more preferably, said amino acid substitution is selected from G113S, G113M, G113F, G113W, G113L, G113T, G113H, and G113Y;
wherein, even more preferably, said amino acid substitution is selected from G113S, G113M, G113F, and G113W.

In one embodiment, the protein, preferably the fatty acid synthase, comprises one or more polypeptide chains, wherein said one or more polypeptide chains comprise
(i) said amino acid sequence encoding a metazoan ketoacyl synthase (KS) domain, preferably said amino acid sequence encoding a murine KS domain; and, optionally,
(ii) said amino acid sequence encoding a bacterial thioesterase ('TesA) domain, preferably said amino acid sequence encoding a thioesterase domain of E. coli; or
   said amino acid sequence encoding a thioreductase (TR) domain, preferably
   said amino acid sequence encoding a bacterial TR domain;
under the proviso that, if the one or more polypeptide chains do not comprise at least one of said amino acid sequence encoding a bacterial thioesterase ('TesA) domain and said amino acid sequence encoding a thioreductase (TR) domain, then the amino acid sequence encoding a metazoan ketoacyl synthase (KS) domain has at least 70%, preferably at least 80%, more preferably at least 90%, even more preferably at least 95% sequence identity to the amino acid sequence of SEQ ID NO: 1 and comprises an amino acid substitution in the KS domain at a position corresponding to G113, S117, or T196 of the amino acid sequence of SEQ ID NO: 1; wherein, preferably, said amino acid substitution is selected from G113S, G113M, G113F, G113W, G113L, G113I, G113T, G113H, G113Y, S117R, S117Y, and T196L;
wherein, more preferably, said amino acid substitution is selected from G113S, G113M, G113F, G113W, G113L, G113T, G113H, and G113Y;
wherein, even more preferably, said amino acid substitution is selected from G113S, G113M, G113F, and G113W.

In one embodiment, the protein is a protein involved in fatty acid synthesis, particularly having fatty acid synthase activity. The protein may comprise, for example, two, three, four or more polypeptide chains. For example, each polypeptide chain may encode a domain of the protein. In one embodiment, said protein comprises a polypeptide chain encoding an amino acid sequence encoding a metazoan ketoacyl synthase (KS) domain and comprises a polypeptide chain which encodes an amino acid sequence encoding a bacterial thioesterase ('TesA) domain or which encodes an amino acid sequence encoding a thioreductase (TR) domain. Advantageously, the protein of the invention allows for precise control over the chain length of the hydrocarbons produced, enabling the synthesis of specific short-chain and medium-chain fatty acids, as well as alcohols or aldehydes thereof. Thus, the protein of the invention efficiently produces fatty acids, fatty alcohols, and/or fatty aldehydes, e.g. for industrial applications where specific chain lengths are required for different uses such as biofuels, lubricants, and pharmaceuticals. In one embodiment, the protein comprises, particularly the one or more polypeptide chains comprise, said amino acid sequence encoding a metazoan ketoacyl synthase (KS) domain and said amino acid sequence encoding a bacterial thioesterase ('TesA) domain. In one embodiment, the protein comprises, particularly the one or more polypeptide chains comprise, an amino acid sequence encoding a metazoan KS domain having at least 70%, preferably at least 80%, more preferably at least 90%, even more preferably at least 95% sequence identity to the amino acid sequence of SEQ ID NO: 1; and the protein comprises, particularly the one or more polypeptide chains comprise, an amino acid sequence encoding a bacterial 'TesA domain having at least 70%, preferably at least 80%, more preferably at least 90%, even more preferably at least 95% sequence identity to the amino acid sequence of SEQ ID NO: 2. In one embodiment, the protein comprises, particularly the one or more polypeptide chains comprise, said amino acid sequence encoding a metazoan ketoacyl synthase (KS) domain and said amino acid sequence encoding a thioreductase (TR) domain. In one embodiment, the protein comprises, particularly the one or more polypeptide chains comprise, an amino acid sequence encoding a metazoan KS domain having at least 70%, preferably at least 80%, more preferably at least 90%, even more preferably at least 95% sequence identity to the amino acid sequence of SEQ ID NO: 1; and the protein comprises, particularly the one or more polypeptide chains comprise, an amino acid sequence encoding a TR domain having at least 70%, preferably at least 80%, more preferably at least 90%, even more preferably at least 95% sequence identity to the amino acid sequence of SEQ ID NO: 3, 4, or 5. In one embodiment, said protein produces a fatty acid, a fatty alcohol, and/or a fatty aldehyde. In one embodiment, said protein produces a fatty acid having a chain length of Cs or less and/or having a chain length of C₁₀-C₁₄, or an alcohol and/or aldehyde thereof. In one embodiment, said protein produces SCFAs and/or MCFAs.

The amino acid sequences of SEQ ID NO: 1-5 and 35 are as follows:
**SEQ ID NO: 1** [KS]: 
MEEVVIAGMSGKLPESENLQEFWANLIGGVDMVTDDDRRWKAGLYGLPKRSGKLKDLSKF
DASFFGVHPKQAHTMDPQLRLLLEVSYEAIVDGGINPASLRGTNTGVWVGVSGSEASEAL
SRDPETLLGYSMVGCQRAMMANRLSFFFDFKGPSIALDTACSSSLLALQNAYQAIRSGEC
PAALVGGINLLLKPNTSVQFMKLGMLSPDGTCRSFDDSGSGYCRSEAVVAVLLTKKSLAR
RVYATILNAGTNTDGSKEQGVTFPSGEVQEQLICSLYQPAGLAPESLEYIEAHGTGTKVG
DPQELNGITRSLCAFRQAPLLIGSTKSNMGHPEPASGLAALTKVLLSLEHGVWAPNLHFH
NPNPEIPALLDGRLQVVDRPLPVRGGNVGINSFGFGGSNVHVILQP.
**SEQ ID NO: 2** ['TesA]: 
ADTLLILGDSLSAGYRMSASAAWPALLNDKWQSKTSVVNASISGDTSQQGLARLPALLKQ
HQPRWVLVELGGNDGLRGFQPQQTEQTLRQILQDVKAANAEPLLMQIRLPANYGRRYNEA
FSAIYPKLAKEFDVPLLPFFMEEVYLKPQWMQDDGIHPNRDAQPFIADWMAKQLQPLVNH
DS.
**SEQ ID NO:** 3 [TR_Mphl]: 
TARRPGDGRPTFAGVHGDDAAEVHARDLTLDRFLDDATLAAATALPGPAPEIRTVLLTGA
TGFLGRYLALEWLERMAMVGGTLICLVRGRDDAAARARLDQIFDSGDPELLRHYRELADR
HLEVIAGDKSDAVLGLDRRTWQRLADTVDLIVDPAALVNHVLPYRELFGPNVVGTAELIR
LALTGRLKPYLYTSTIAVGAGIAPGQFTEDADIRQISATRTLDDSYANGYATSKWAGEVL
LREAHDLCGLPVAVFRCDMILADTSYAGQLNVPDMFTRLILSLVATGIAPLSFYELDAAG
HRQRAHYDGLPVEFVAEAVSALGLDVAEDGGFATYHVMNPYDDGIGLDEFVDWLTGAGYP
IERVNDYGTWFQRFETAIRGLPERQRQASLLPLLHNYQRPQPPIRGSAAPTDRFRSAVQD
AKIGPDKDIPHITPEVIVKYVTDLRLLGLL.
**SEQ ID NO:** 4 [TR_Msme]: 
AQRTAGDRRPSFTTVHGADATEIRASELTLDKFIDAETLRAAPGLPKVTTEPRTVLLSGA
NGWLGRFLTLQWLERLAPVGGTLITIVRGRDDAAARARLTQAYDTDPELSRRFAELADRH
LRVVAGDIGDPNLGLTPEIWHRLAAEVDLVVHPAALVNHVLPYRQLFGPNVVGTAEVIKL
ALTERIKPVTYLSTVSVAMGIPDFEEDGDIRTVSPVRPLDGGYANGYGNSKWAGEVLLRE
AHDLCGLPVATFRSDMILAHPRYRGQVNVPDMFTRLLLSLLITGVAPRSFYIGDGERPRA
HYPGLTVDFVAEAVTTLGAQQREGYVSYDVMNPHDDGISLDVFVDWLIRAGHPIDRVDDY
DDWVRRFETALTALPEKRRAQTVLPLLHAFRAPQAPLRGAPEPTEVFHAAVRTAKVGPGD
IPHLDEALIDKYIRDLREFGLI.
**SEQ ID NO:** 5 [TR_Noti]: 
SERDSGSRPTAATVHGDDGLLRADDLALEAFLDPATLDAAAHLPSALEPPRTVLLTGANGYL
GRFLALEWLQRLDVSGGTLICLIRGSDADSARRRLDAVFATGDPELEAHYRELAERRLRVLP
GDIGEPNLGLREQDWRDLAETVDLIVHPAALVNHVLPYAQLFGPNVVGTAEVIRLALTSRLK
PVTYLSTVAVSAGIDPETFTEDGDIREISPVRRLDDGYANGYGNSKWAGEVLLRNAHDRFGL
PVAVFRSDMILAHSRYAGQLNVPDMFTRLLLSVLATGLAPGSFHDAHGERHRAHYDGLPAD
FTAAAVTTLGSRVTSGYETYDVLNPHDDGISLDTFVDWLIEAGHPIDRIDDYAEWFARFDTA
LRALPEHQRQHSLLPLLHAYRRPTPPLHGVALPAKHFRAAVQQAKLGPDGDIPHVTRELIEK
YASDLRLLGLIQ.
SEQ **ID** NO: **35** [TE_WT]:

LSTLLVNPEGPTLTQLNSVQSSERPLFLVHPIEGSTTVFHSLAAKLSVPTYGLQCTQAAPLDSI
PNLAAYYIDCIKQVQPEGPYRIAGYSFGACVAFEMCSQLQAQQGPAPTHNNLFLFDGSHTYV
LAYTQSYRAKMTPGCEAEAEAEALCFFIKQFLDVEHSKVLEALLPLKSLEDRVAASVDLITKS
HHSLDRRELSFAAVSFYHKLRAADQYKPKAKYHGNVTLLRAKTGGTYGEDLGADYNLSQVC
DGKVSVHIIEGDHRTLLEGSGLESIINIIHSSLAEPRVSVREG.

**In** one embodiment, the ketoacyl synthase (KS) domain is derived from a metazoan fatty acid synthase, e.g. a murine fatty acid synthase. The bacterial thioesterase domain may be a truncated thioesterase domain, particularly a biologically functional truncated thioesterase domain, such as a truncated thioesterase domain which has been truncated by removing an N-terminal end which mediates membrane binding to solubilize said thioesterase domain. **In** this context, "biologically functional" is meant to be understood as referring to a truncated version which maintains the enzymatic activity. For example, a biologically functional truncated thioesterase domain may be a domain which maintains the enzymatic activity of the untruncated thioesterase domain. The abbreviation "TesA", as used herein, relates to a bacterial thioesterase, optionally to a truncated bacterial thioesterase such as a truncated thioesterase of E.coli. For example, when referring to a 'TesA domain, a bacterial thioesterase domain is meant. In one embodiment, said amino acid sequence encoding a metazoan KS domain has at least 70%, preferably at least 80%, more preferably at least 90%, even more preferably at least 95% sequence identity, such as at least 98% or 99% sequence identity, to the amino acid sequence of SEQ ID NO: 1. In one embodiment, said amino acid sequence encoding a bacterial 'TesA domain has at least 70%, preferably at least 80%, more preferably at least 90%, even more preferably at least 95% sequence identity, such as 98% sequence identity or 99% sequence identity, to the amino acid sequence of SEQ ID NO: 2. In one embodiment, said amino acid sequence encoding a TR domain has at least 70%, preferably at least 80%, more preferably at least 90%, even more preferably at least 95% sequence identity, such as 98% sequence identity or 99% sequence identity, to the amino acid sequence of SEQ ID NO: 3, 4, or 5.

In one embodiment, the amino acid sequence encoding a metazoan KS domain has at least 70%, preferably at least 80%, more preferably at least 90%, even more preferably at least 95% sequence identity, such as at least 98% or 99% sequence identity, to the amino acid sequence of SEQ ID NO: 1 and comprises an amino acid substitution in the KS domain at a position corresponding to G113, S117, or T196 of the amino acid sequence of SEQ ID NO: 1. For example, the amino acid sequence encoding a metazoan KS domain may have at least 70%, preferably at least 80%, more preferably at least 90%, even more preferably at least 95% sequence identity, such as at least 98% or 99% sequence identity, to the amino acid sequence of SEQ ID NO: 1 and may comprise an amino acid substitution in the KS domain at a position corresponding to G113 of the amino acid sequence of SEQ ID NO: 1, an amino acid substitution in the KS domain at a position corresponding to S117 of the amino acid sequence of SEQ ID NO: 1, and/or an amino acid substitution in the KS domain at a position corresponding to T196 of the amino acid sequence of SEQ ID NO: 1. In one embodiment, the amino acid sequence encoding a metazoan KS domain has at least 70%, preferably at least 80%, more preferably at least 90%, even more preferably at least 95% sequence identity, such as at least 98% or 99% sequence identity, to the amino acid sequence of SEQ ID NO: 1 and comprises one or more amino acid substitutions in the KS domain at one or more positions corresponding to G113, S117, and T196 of the amino acid sequence of SEQ ID NO: 1.

In a preferred embodiment, said amino acid substitution in the KS domain, particularly in the metazoan KS domain having at least 70%, preferably at least 80%, more preferably at least 90%, even more preferably at least 95% sequence identity, such as at least 98% or 99% sequence identity, to the amino acid sequence of SEQ ID NO: 1, is selected from G113S, G113M, G113F, G113W, G113L, G113I, G113T, G113H, G113Y, S117R, S117Y, and T196L. In a more preferred embodiment, said amino acid substitution in the KS domain, particularly in the metazoan KS domain having at least 70%, preferably at least 80%, more preferably at least 90%, even more preferably at least 95% sequence identity, such as at least 98% or 99% sequence identity, to the amino acid sequence of SEQ ID NO: 1, is selected from G113S, G113M, G113F, G113W, G113L, G113T, G113H, and G113Y. In an even more preferred embodiment, said amino acid substitution in the KS domain, particularly in the metazoan KS domain having at least 70%, preferably at least 80%, more preferably at least 90%, even more preferably at least 95% sequence identity, such as at least 98% or 99% sequence identity, to the amino acid sequence of SEQ ID NO: 1, is selected from G113S, G113M, G113F, and G113W.

In one embodiment, the amino acid sequence encoding a metazoan KS domain has at least 70%, preferably at least 80%, more preferably at least 90%, even more preferably at least 95% sequence identity, such as at least 98% or 99% sequence identity, to the amino acid sequence of SEQ ID NO: 1 and comprises an amino acid substitution in the KS domain at a position corresponding to G113 of the amino acid sequence of SEQ ID NO: 1, preferably an amino acid substitution selected from G113S, G113M, G113F, G113W, G113L, G113I, G113T, G113H, and G113Y.

As used herein, the term "at a position corresponding to" means the respective position in the sequence of the respective SEQ ID No. In one embodiment, in related polypeptide chains, the respective position has another relative position number. In one embodiment, an equivalent substitution is determined by comparing a position in both sequences, which may be aligned for the purpose of comparison. The relative position of the amino acid may vary due to different length of the related polypeptide, or deletions or additions of amino acids in the related polypeptide. As used herein, the term "percent (%) identity" refers to sequence identity between two amino acid sequences. Identity can be determined by comparing a position in both sequences, which may be aligned for the purpose of comparison. In calculating percent identity, the sequences being compared are typically aligned in a way that gives the largest match between the sequences. When an equivalent position in the compared sequences is occupied by the same amino acid, the molecules are considered to be identical at that position. Generally, a person skilled in the art is aware of the fact that some amino acid exchanges in the amino acid sequence of a protein do not have an influence on the (secondary or tertiary) structure, function and/or activity of that protein. Amino acid sequences with such "neutral" amino acid exchanges as compared to the amino acid sequences disclosed herein fall within the scope of the present invention. It is meant to be understood that, when referring to an amino acid sequence having a particular percentage of sequence identity, such as at least 70% sequence identity or at least 80% sequence identity, to a sequence of a SEQ ID NO:, amino acid sequences having 100% sequence identity to the respective SEQ ID NO: are also encompassed. For example, when referring to an amino acid sequence encoding a bacterial 'TesA domain having at least 70% sequence identity to the amino acid sequence of SEQ ID NO: 2, an amino acid sequence encoding a bacterial 'TesA domain having 100% sequence identity to the amino acid sequence of SEQ ID NO: 2 is also meant to be directly and unambiguously disclosed.

In one embodiment, the amino acid sequence encoding a 'TesA domain has at least 70%, preferably at least 80%, more preferably at least 90%, even more preferably at least 95% sequence identity, such as at least 98% or 99% sequence identity, to the amino acid sequence of SEQ ID NO: 2 and comprises one or more amino acid substitutions in the 'TesA domain at a position corresponding to L109, M141, E142, Y145, and/or L146 of the amino acid sequence of SEQ ID NO: 2. In a preferred embodiment, said one or more amino acid substitutions in the 'TesA domain, said 'TesA domain having at least 70%, preferably at least 80%, more preferably at least 90%, even more preferably at least 95% sequence identity, such as at least 98% or 99% sequence identity, to the amino acid sequence of SEQ ID NO: 2, are selected from L109P, M141L, E142D, Y145G, L146K, and combinations thereof. In a more preferred embodiment, said one or more amino acid substitutions in the 'TesA domain, said 'TesA domain having at least 70%, preferably at least 80%, more preferably at least 90%, even more preferably at least 95% sequence identity, such as at least 98% or 99% sequence identity, to the amino acid sequence of SEQ ID NO: 2, are amino acid substitution L109P or amino acid substitutions M141L, E142D, Y145G, and L146K.

In one embodiment, the TR domain is a TR domain of a *Mycobacterium* sp., a *Nocardia* sp., *a Tsukamurella* sp., a *Neurospora* sp., a *Stigmatella* sp., or *Methanobrevibacter* sp. In one embodiment, the TR domain is a TR domain of a carboxylic acid reductase (CAR) or of a hybrid polyketide synthase non-ribosomal peptide synthetase, preferably a TR domain of a CAR. In a preferred embodiment, the TR domain is a TR domain of *Mycobacterium marinum, Mycobacterium phlei, Mycobacterium smegmatis, Mycobacterium tuberculosis, Nocardia iowensis, Nocardia otitidiscaviarum, Tsukamurella paurometabola, Neurospora crassa, Stigmatella aurantiaca,* or *Methanobrevibacter ruminantium.* In a more preferred embodiment, the TR domain is a TR domain of *Mycobacterium phlei, Mycobacterium smegmatis,* or *Nocardia otitidiscaviarum,* optionally a TR domain of a CAR of *Mycobacterium phlei, Mycobacterium smegmatis,* or *Nocardia otitidiscaviarum.* In one embodiment, the amino acid sequence encoding a TR domain has at least 70%, preferably at least 80%, more preferably at least 90%, even more preferably at least 95% sequence identity, such as at least 98% or 99% sequence identity, to the amino acid sequence of SEQ ID NO: 3, 4, or 5. The term "TR1", as used herein, may relate to TR_Mphl, e.g. having SEQ ID NO: 3. The term "TR2", as used herein, may relate to TR_Msme, e.g. having SEQ ID NO: 4. The term "TR3", as used herein, may relate to TR_Noti, e.g. having SEQ ID NO: 5.

In one embodiment, said one or more polypeptide chains comprise
(i) an amino acid sequence encoding a metazoan ketoacyl synthase (KS) domain, preferably an amino acid sequence encoding a murine KS domain; and
(ii) an amino acid sequence encoding a bacterial thioesterase ('TesA) domain, preferably an amino acid sequence encoding a thioesterase domain of E. coli; or an amino acid sequence encoding a thioreductase (TR) domain, preferably an amino acid sequence encoding a bacterial TR domain;
or said one or more polypeptide chains comprise
(i) an amino acid sequence encoding a metazoan ketoacyl synthase (KS) domain, preferably an amino acid sequence encoding a murine KS domain; wherein the amino acid sequence encoding a metazoan KS domain has at least 70%, preferably at least 80%, more preferably at least 90%, even more preferably at least 95% sequence identity to the amino acid sequence of SEQ ID NO: 1 and comprises an amino acid substitution in the KS domain at a position corresponding to G113, S117, or T196 of the amino acid sequence of SEQ ID NO: 1;
   wherein, preferably, said amino acid substitution is selected from G113S, G113M, G113F, G113W, G113L, G113I, G113T, G113H, G113Y, S117R, S117Y, and T196L;
   wherein, more preferably, said amino acid substitution is selected from G113S, G113M, G113F, G113W, G113L, G113T, G113H, and G113Y;
   wherein, even more preferably, said amino acid substitution is selected from G113S, G113M, G113F, and G113W; and
(ii) an amino acid sequence encoding a thioesterase domain, preferably a metazoan thioesterase domain, more preferably a thioesterase domain having at least 70%, preferably at least 80%, more preferably at least 90%, even more preferably at least 95% sequence identity to the amino acid sequence of SEQ ID NO: 35.

In one embodiment, said one or more polypeptide chains comprise
(i) said amino acid sequence encoding a metazoan ketoacyl synthase (KS) domain, preferably said amino acid sequence encoding a murine KS domain; and
(ii) said amino acid sequence encoding a bacterial thioesterase ('TesA) domain, preferably said amino acid sequence encoding a thioesterase domain of E. coli; or said amino acid sequence encoding a thioreductase (TR) domain, preferably said amino acid sequence encoding a bacterial TR domain.

In one embodiment, said one or more polypeptide chains comprise
(i) said amino acid sequence encoding a metazoan ketoacyl synthase (KS) domain, preferably said amino acid sequence encoding a murine KS domain;
   wherein the amino acid sequence encoding a metazoan KS domain has at least 70%, preferably at least 80%, more preferably at least 90%, even more preferably at least 95% sequence identity to the amino acid sequence of SEQ ID NO: 1 and comprises an amino acid substitution in the KS domain at a position corresponding to G113, S117, or T196 of the amino acid sequence of SEQ ID NO: 1;
   wherein, preferably, said amino acid substitution is selected from G113S, G113M, G113F, G113W, G113L, G113I, G113T, G113H, G113Y, S117R, S117Y, and T196L;
   wherein, more preferably, said amino acid substitution is selected from G113S, G113M, G113F, G113W, G113L, G113T, G113H, and G113Y;
   wherein, even more preferably, said amino acid substitution is selected from G113S, G113M, G113F, and G113W; and
(ii) said amino acid sequence encoding a thioesterase domain, preferably a metazoan thioesterase domain, more preferably a thioesterase domain having at least 70%, preferably at least 80%, more preferably at least 90%, even more preferably at least 95% sequence identity to the amino acid sequence of SEQ ID NO: 35.

In one embodiment, said amino acid sequence encoding a thioesterase domain is an amino acid sequence encoding a metazoan thioesterase domain, preferably an amino acid sequence encoding a murine thioesterase domain, more preferably an amino acid sequence encoding a thioesterase domain having at least 70%, preferably at least 80%, more preferably at least 90%, even more preferably at least 95%, such as 98% or 99%, optionally 100%, sequence identity to the amino acid sequence of SEQ ID NO: 35.

In one embodiment, the protein comprises, particularly the one or more polypeptide chains comprise,
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1; and
   an amino acid sequence encoding a 'TesA domain, preferably having at least 70%, preferably at least 80%, more preferably at least 90%, even more preferably at least 95% sequence identity, such as at least 98% or 99% sequence identity, to the amino acid sequence of SEQ ID NO: 2; or
- an amino acid sequence encoding a metazoan KS domain having at least 70%, preferably at least 80%, more preferably at least 90%, even more preferably at least 95% sequence
   identity, such as at least 98% or 99% sequence identity, to the amino acid sequence of SEQ ID NO: 1; and
   an amino acid sequence encoding a 'TesA domain having at least 70%, preferably at least 80%, more preferably at least 90%, even more preferably at least 95% sequence identity, such as at least 98% or 99% sequence identity, to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1; and
   an amino acid sequence encoding a 'TesA domain having at least 70%, preferably at least 80%, more preferably at least 90%, even more preferably at least 95% sequence identity, such as at least 98% or 99% sequence identity, to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113S; and
   an amino acid sequence encoding a 'TesA domain, preferably having at least 70%, preferably at least 80%, more preferably at least 90%, even more preferably at least 95% sequence identity, such as at least 98% or 99% sequence identity, to the amino acid sequence of SEQ ID NO: 2; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113S; and
   an amino acid sequence encoding a 'TesA domain having at least 70%, preferably at least 80%, more preferably at least 90%, even more preferably at least 95% sequence identity, such as at least 98% or 99% sequence identity, to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113S; and
   an amino acid sequence encoding a 'TesA domain having at least 70%, preferably at least 80%, more preferably at least 90%, even more preferably at least 95% sequence identity, such as at least 98% or 99% sequence identity, to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113M; and
   an amino acid sequence encoding a 'TesA domain, preferably having at least 70%, preferably at least 80%, more preferably at least 90%, even more preferably at least 95% sequence identity, such as at least 98% or 99% sequence identity, to the amino acid sequence of SEQ ID NO: 2; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113M; and
   an amino acid sequence encoding a 'TesA domain having at least 70%, preferably at least 80%, more preferably at least 90%, even more preferably at least 95% sequence identity, such as at least 98% or 99% sequence identity, to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113M; and
   an amino acid sequence encoding a 'TesA domain having at least 70%, preferably at least 80%, more preferably at least 90%, even more preferably at least 95% sequence identity, such as at least 98% or 99% sequence identity, to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113F; and
   an amino acid sequence encoding a 'TesA domain, preferably having at least 70%, preferably at least 80%, more preferably at least 90%, even more preferably at least 95% sequence identity, such as at least 98% or 99% sequence identity, to the amino acid sequence of SEQ ID NO: 2; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113F; and
   an amino acid sequence encoding a 'TesA domain having at least 70%, preferably at least 80%, more preferably at least 90%, even more preferably at least 95% sequence identity, such as at least 98% or 99% sequence identity, to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113F; and
   an amino acid sequence encoding a 'TesA domain having at least 70%, preferably at least 80%, more preferably at least 90%, even more preferably at least 95% sequence identity, such as at least 98% or 99% sequence identity, to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113W; and
   an amino acid sequence encoding a 'TesA domain, preferably having at least 70%, preferably at least 80%, more preferably at least 90%, even more preferably at least 95% sequence identity, such as at least 98% or 99% sequence identity, to the amino acid sequence of SEQ ID NO: 2; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113W; and
   an amino acid sequence encoding a 'TesA domain having at least 70%, preferably at least 80%, more preferably at least 90%, even more preferably at least 95% sequence identity, such as at least 98% or 99% sequence identity, to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113W; and
   an amino acid sequence encoding a 'TesA domain having at least 70%, preferably at least 80%, more preferably at least 90%, even more preferably at least 95% sequence identity, such as at least 98% or 99% sequence identity, to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113T; and
   an amino acid sequence encoding a 'TesA domain, preferably having at least 70%, preferably at least 80%, more preferably at least 90%, even more preferably at least 95% sequence identity, such as at least 98% or 99% sequence identity, to the amino acid sequence of SEQ ID NO: 2; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113L; and
   an amino acid sequence encoding a 'TesA domain, preferably having at least 70%, preferably at least 80%, more preferably at least 90%, even more preferably at least 95% sequence identity, such as at least 98% or 99% sequence identity, to the amino acid sequence of SEQ ID NO: 2; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113H; and
   an amino acid sequence encoding a 'TesA domain, preferably having at least 70%, preferably at least 80%, more preferably at least 90%, even more preferably at least 95% sequence identity, such as at least 98% or 99% sequence identity, to the amino acid sequence of SEQ ID NO: 2; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113Y; and
   an amino acid sequence encoding a 'TesA domain, preferably having at least 70%, preferably at least 80%, more preferably at least 90%, even more preferably at least 95% sequence identity, such as at least 98% or 99% sequence identity, to the amino acid sequence of SEQ ID NO: 2; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113S; and
   an amino acid sequence encoding a TR domain having at least 70%, preferably at least 80%, more preferably at least 90%, even more preferably at least 95% sequence identity, such as at least 98% or 99% sequence identity, to the amino acid sequence of SEQ ID NO: 3, 4, or 5; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113M; and
   an amino acid sequence encoding a TR domain having at least 70%, preferably at least 80%, more preferably at least 90%, even more preferably at least 95% sequence identity, such as at least 98% or 99% sequence identity, to the amino acid sequence of SEQ ID NO: 3, 4, or 5; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113F; and
   an amino acid sequence encoding a TR domain having at least 70%, preferably at least 80%, more preferably at least 90%, even more preferably at least 95% sequence identity, such as at least 98% or 99% sequence identity, to the amino acid sequence of SEQ ID NO: 3, 4, or 5; or
- an amino acid sequence encoding a metazoan KS domain having at least 70%, preferably at least 80%, more preferably at least 90%, even more preferably at least 95% sequence identity, such as at least 98% or 99% sequence identity, to the amino acid sequence of SEQ ID NO: 1 and comprising the amino acid substitution G113W; and
   an amino acid sequence encoding a TR domain having at least 70%, preferably at least 80%, more preferably at least 90%, even more preferably at least 95% sequence identity, such as at least 98% or 99% sequence identity, to the amino acid sequence of SEQ ID NO: 3, 4, or 5.

For example, a protein for producing a fatty alcohol and/or fatty aldehyde may comprise an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113W; and
an amino acid sequence encoding a TR domain having at least 70%, preferably at least 80%, more preferably at least 90%, even more preferably at least 95% sequence identity to the amino acid sequence of SEQ ID NO: 3, 4, or 5.

In one embodiment, said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
SEQ ID NO: 6 [mFASWT-'TesA],
SEQ ID NO: 7 [mFASWT-'TesAL109P],
SEQ ID NO: 8 [mFASWT-'TesA4x],
SEQ ID NO: 9 [mFASG113S-TE_WT],
SEQ ID NO: 10 [mFASG113S-'TesA],
SEQ ID NO: 11 [mFASG113S-"TesAL109P],
SEQ ID NO: 12 [mFASG113S-'TesA4x],
SEQ ID NO: 13 [mFASG113M-TE_WT],
SEQ ID NO: 14 [mFASG113M-'TesA],
SEQ ID NO: 15 [mFASG113M-'TesAL109P],
SEQ ID NO: 16 [mFASG113M-'TesA4x],
SEQ ID NO: 17 [mFASG113F-TE_WT],
SEQ ID NO: 18 [mFASG113F-'TesA],
SEQ ID NO: 19 [mFASG113F-'TesAL109P],
SEQ ID NO: 20 [mFASG113F-'TesA4x],
SEQ ID NO: 21 [mFASG113W-TE_WT],
SEQ ID NO: 22 [mFASG113W-'TesA],
SEQ ID NO: 23 [mFASG113W-'TesAL109P],
SEQ ID NO: 24 [mFASG113W-'TesA4x],
SEQ ID NO: 31 [mFASG113T-'TesA],
SEQ ID NO: 32 [mFASG113L-'TesA],
SEQ ID NO: 33 [mFASG113H-'TesA], and
SEQ ID NO: 34 [mFASG113Y-'TesA];
wherein, preferably, said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
SEQ ID NO: 10 [mFASG113S-'TesA],
SEQ ID NO: 11 [mFASG113S-"TesAL109P],
SEQ ID NO: 12 [mFASG113S-'TesA4x],
SEQ ID NO: 13 [mFASG113M-TE_WT],
SEQ ID NO: 14 [mFASG113M-'TesA],
SEQ ID NO: 15 [mFASG113M-'TesAL109P],
SEQ ID NO: 16 [mFASG113M-'TesA4x],
SEQ ID NO: 18 [mFASG113F-'TesA],
SEQ ID NO: 19 [mFASG113F-'TesAL109P],
SEQ ID NO: 20 [mFASG113F-'TesA4x],
SEQ ID NO: 22 [mFASG113W-'TesA],
SEQ ID NO: 23 [mFASG113W-'TesAL109P],
SEQ ID NO: 24 [mFASG113W-'TesA4x],
SEQ ID NO: 31 [mFASG113T-'TesA],
SEQ ID NO: 32 [mFASG113L-'TesA],
SEQ ID NO: 33 [mFASG113H-'TesA], and
SEQ ID NO: 34 [mFASG113Y-'TesA];
wherein, optionally, said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
SEQ ID NO: 10 [mFASG113S-'TesA],
SEQ ID NO: 12 [mFASG113S-'TesA4x],
SEQ ID NO: 14 [mFASG113M-'TesA], and
SEQ ID NO: 16 [mFASG113M-'TesA4x],
such as an amino acid sequence selected from
SEQ ID NO: 12 [mFASG113S-'TesA4x] and
SEQ ID NO: 14 [mFASG113M-'TesA].

The proteins described herein, particularly proteins comprising a metazoan KS domain and a bacterial thioesterase domain, are highly advantageous in that they produce specific fatty acids, such as SCFA and MCFA.

In one embodiment, said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
SEQ ID NO: 25 [mFAS-TR2],
SEQ ID NO: 26 [mFAS-TR1],
SEQ ID NO: 27 [mFAS-TR3],
SEQ ID NO: 28 [mFASG113W-TR2],
SEQ ID NO: 29 [mFASG113W-TR1], and
SEQ ID NO: 30 [mFASG113W-TR3];
wherein, preferably, said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
SEQ ID NO: 25 [mFAS-TR2]
SEQ ID NO: 28 [mFASG113W-TR2],
SEQ ID NO: 29 [mFASG113W-TR1], and
SEQ ID NO: 30 [mFASG113W-TR3];
wherein, more preferably, said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
SEQ ID NO: 28 [mFASG113W-TR2],
SEQ ID NO: 29 [mFASG113W-TR1], and
SEQ ID NO: 30 [mFASG113W-TR3];
wherein, even more preferably, said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
SEQ ID NO: 28 [mFASG113W-TR2].

The proteins described herein, particularly proteins comprising a metazoan KS domain and a thioreductase domain, are highly advantageous in that they produce specific fatty alcohols and/or fatty aldehydes, such as SCFA and/or MCFA alcohols or SCFA and/or MCFA aldehydes.

For example, proteins comprising an amino acid sequence having at least 70%, preferably at least 80%, more preferably at least 90%, even more preferably at least 95% sequence identity, such as at least 98% or 99% sequence identity, to an amino acid sequence selected from
SEQ ID NO: 18 [mFASG113F-'TesA],
SEQ ID NO: 20 [mFASG113F-'TesA4x],
SEQ ID NO: 22 [mFASG113W-'TesA],
SEQ ID NO: 24 [mFASG113W-'TesA4x],
SEQ ID NO: 33 [mFASG113H-'TesA], and
SEQ ID NO: 34 [mFASG113Y-'TesA] may be used for producing a Cs fatty acid.

Advantageously, said variants efficiently produce C₈ fatty acids.

For example, proteins comprising an amino acid sequence having at least 70%, preferably at least 80%, more preferably at least 90%, even more preferably at least 95% sequence identity, such as at least 98% or 99% sequence identity, to an amino acid sequence selected from
SEQ ID NO: 10 [mFASG113S-'TesA],
SEQ ID NO: 17 [mFASG113F-TE_WT],
SEQ ID NO: 21 [mFASG113W-TE_WT],
SEQ ID NO: 22 [mFASG113W-'TesA],
SEQ ID NO: 8 [mFASWT-'TesA4x],
SEQ ID NO: 12 [mFASG113S-'TesA4x],
SEQ ID NO: 14 [mFASG113M-'TesA],
SEQ ID NO: 16 [mFASG113M-'TesA4x],
SEQ ID NO: 18 [mFASG113F-'TesA],
SEQ ID NO: 19 [mFASG113F-'TesAL109P],
SEQ ID NO: 20 [mFASG113F-'TesA4x],
SEQ ID NO: 31 [mFASG113T-'TesA],
SEQ ID NO: 32 [mFASG113L-'TesA],
SEQ ID NO: 33 [mFASG113H-'TesA], and
SEQ ID NO: 34 [mFASG113Y-'TesA], preferably SEQ ID NO: 18 [mFASG113F-'TesA],
may be used for producing a C₁₀ fatty acid. Advantageously, said variants efficiently produce C₁₀ fatty acids.

For example, proteins comprising an amino acid sequence having at least 70%, preferably at least 80%, more preferably at least 90%, even more preferably at least 95% sequence identity, such as at least 98% or 99% sequence identity, to an amino acid sequence selected from
SEQ ID NO: 17 [mFASG113F-TE_WT],
SEQ ID NO: 18 [mFASG113F-'TesA],
SEQ ID NO: 21 [mFASG113W-TE_WT],
SEQ ID NO: 6 [mFASWT-'TesA],
SEQ ID NO: 8 [mFASWT-'TesA4x],
SEQ ID NO: 10 [mFASG113S-'TesA],
SEQ ID NO: 11 [mFASG113S-"TesAL109P],
SEQ ID NO: 12 [mFASG113S-'TesA4x],
SEQ ID NO: 14 [mFASG113M-'TesA],
SEQ ID NO: 15 [mFASG113M-'TesAL109P],
SEQ ID NO: 16 [mFASG113M-'TesA4x],
SEQ ID NO: 19 [mFASG113F-'TesAL109P],
SEQ ID NO: 31 [mFASG113T-'TesA],
SEQ ID NO: 32 [mFASG113L-'TesA],
SEQ ID NO: 33 [mFASG113H-'TesA], and
SEQ ID NO: 34 [mFASG113Y-'TesA],
may be used for producing a C₁₂ fatty acid. Advantageously, said variants efficiently produce C₁₂ fatty acids.

For example, proteins comprising an amino acid sequence having at least 70%, preferably at least 80%, more preferably at least 90%, even more preferably at least 95% sequence identity, such as at least 98% or 99% sequence identity, to an amino acid sequence selected from
SEQ **ID** NO: 9 [mFASG113S-TE_WT],
SEQ ID NO: 13 [mFASG113M-TE_WT],
SEQ ID NO: 17 [mFASG113F-TE_WT],
SEQ ID NO: 21 [mFASG113W-TE_WT],
SEQ ID NO: 6 [mFASWT-'TesA],
SEQ ID NO: 8 [mFASWT-'TesA4x],
SEQ ID NO: 10 [mFASG113S-'TesA],
SEQ ID NO: 11 [mFASG113S-"TesAL109P],
SEQ ID NO: 12 [mFASG113S-'TesA4x],
SEQ ID NO: 14 [mFASG113M-'TesA],
SEQ ID NO: 15 [mFASG113M-'TesAL109P],
SEQ ID NO: 16 [mFASG113M-'TesA4x],
SEQ ID NO: 19 [mFASG113F-'TesAL109P],
SEQ ID NO: 31 [mFASG113T-'TesA],
SEQ ID NO: 32 [mFASG113L-'TesA], and
SEQ ID NO: 33 [mFASG113H-'TesA],
may be used for producing a C₁₄ fatty acid. Advantageously, said variants efficiently produce C₁₄ fatty acids.

For example, proteins comprising an amino acid sequence having at least 70%, preferably at least 80%, more preferably at least 90%, even more preferably at least 95% sequence identity, such as at least 98% or 99% sequence identity, to an amino acid sequence selected from
SEQ ID NO: 9 [mFASG113S-TE_WT],
SEQ ID NO: 13 [mFASG113M-TE_WT],
SEQ ID NO: 17 [mFASG113F-TE_WT],
SEQ ID NO: 21 [mFASG113W-TE_WT],
SEQ ID NO: 6 [mFASWT-'TesA],
SEQ ID NO: 8 [mFASWT-'TesA4x],
SEQ ID NO: 11 [mFASG113S-"TesAL109P], and
SEQ ID NO: 15 [mFASG113M-'TesAL109P],
may be used for producing a C₁₆ fatty acid. Advantageously, said variants efficiently produce C₁₆ fatty acids.

For example, proteins comprising an amino acid sequence having at least 70%, preferably at least 80%, more preferably at least 90%, even more preferably at least 95% sequence identity, such as at least 98% or 99% sequence identity, to an amino acid sequence selected from
SEQ ID NO: 9 [mFASG113S-TE_WT],
SEQ ID NO: 13 [mFASG113M-TE_WT],
SEQ ID NO: 17 [mFASG113F-TE_WT],
SEQ ID NO: 21 [mFASG113W-TE_WT],
SEQ ID NO: 7 [mFASWT-'TesAL109P],
SEQ ID NO: 11 [mFASG113S-'TesAL109P],
SEQ ID NO: 12 [mFASG113S-'TesA4x], and
SEQ ID NO: 15 [mFASG113M-'TesAL109P],
may be used for producing a C₁₈ fatty acid. Advantageously, said variants efficiently produce C₁₈ fatty acids.

For example, proteins comprising an amino acid sequence having at least 70%, preferably at least 80%, more preferably at least 90%, even more preferably at least 95% sequence identity, such as at least 98% or 99% sequence identity, to an amino acid sequence selected from
SEQ ID NO: 28 [mFASG113W-TR2],
SEQ ID NO: 29 [mFASG113W-TR1], and
SEQ ID NO: 30 [mFASG113W-TR3],
may be used for producing a C₈, C₁₀, and/or C₁₂ fatty alcohol and/or C₈, C₁₀, and/or C₁₂ fatty aldehyde. Advantageously, said variants efficiently produce C₈, C₁₀, and/or C₁₂ fatty alcohols and/or C₈, C₁₀, and/or C₁₂ fatty aldehydes.

For example, proteins comprising an amino acid sequence having at least 70%, preferably at least 80%, more preferably at least 90%, even more preferably at least 95% sequence identity, such as at least 98% or 99% sequence identity, to an amino acid sequence of SEQ ID NO: 26 may be used for producing a C₁₆, C₁₈, and/or C₂₀ aldehyde. Advantageously, said variant efficiently produces C₁₆, C₁₈, and/or C₂₀ aldehyde(s).

Herein, when a term is mentioned in brackets, particularly square brackets, in the context of a SEQ ID NO, the respective term in brackets relates to the name of the sequence encoded by the respective SEQ ID NO. The respective term in brackets merely corresponds to the name of the encoded sequence, and said term in brackets may thus be omitted when referring to the respective SEQ ID NO. For example, the expression "[mFAS-TR2]" in the context of SEQ ID NO: 25 indicates that SEQ ID NO: 25 encodes a mFAS-TR2 sequence, particularly a sequence comprising mFAS and TR2. Accordingly, a feature reciting, for example, "an amino acid sequence selected from
SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 31, SEQ ID NO: 32, SEQ ID NO: 33, and
SEQ ID NO: 34" is identical to a feature reciting "an amino acid sequence selected from
SEQ ID NO: 6 [mFASWT-'TesA],
SEQ ID NO: 7 [mFASWT-'TesAL109P],
SEQ ID NO: 8 [mFASWT-'TesA4x],
SEQ ID NO: 9 [mFASG113S-TE_WT],
SEQ ID NO: 10 [mFASG113S-'TesA],
SEQ ID NO: 11 [mFASG113S-"TesAL109P],
SEQ ID NO: 12 [mFASG113S-'TesA4x],
SEQ ID NO: 13 [mFASG113M-TE_WT],
SEQ ID NO: 14 [mFASG113M-'TesA],
SEQ ID NO: 15 [mFASG113M-'TesAL109P],
SEQ ID NO: 16 [mFASG113M-'TesA4x],
SEQ ID NO: 17 [mFASG113F-TE_WT],
SEQ ID NO: 18 [mFASG113F-'TesA],
SEQ ID NO: 19 [mFASG113F-'TesAL109P],
SEQ ID NO: 20 [mFASG113F-'TesA4x],
SEQ ID NO: 21 [mFASG113W-TE_WT],
SEQ ID NO: 22 [mFASG113W-'TesA],
SEQ ID NO: 23 [mFASG113W-'TesAL109P],
SEQ ID NO: 24 [mFASG113W-'TesA4x],
SEQ ID NO: 31 [mFASG113T-'TesA],
SEQ ID NO: 32 [mFASG113L-'TesA],
SEQ ID NO: 33 [mFASG113H-'TesA], and
SEQ ID NO: 34 [mFASG113Y-'TesA]".

In one embodiment, the protein does not comprise a thioesterase (TE) domain. In one embodiment, the protein is a fatty acid synthase in which the TE domain is replaced by the 'TesA domain, as defined herein, or the TR domain, as defined herein.

In a further aspect, the present invention relates to a nucleic acid molecule encoding a protein, said protein being as defined herein. In one embodiment, the nucleic acid molecule of the present invention further comprises a vector nucleic acid sequence, preferably an expression vector sequence, and/or a promoter nucleic acid sequence and a terminator nucleic acid sequence. In one embodiment, the nucleic acid molecule is in the form of a nucleic acid expression construct, such as an expression cassette comprising a nucleic acid molecule according to the invention, or an expression vectors comprising a nucleic acid molecule according to the invention. For example, a nucleic acid expression construct may comprise regulatory sequences, such as promoter and terminator sequences, which are operatively linked with the nucleic acid sequence coding for the protein of the invention. The nucleic acid expression construct may further comprise 5' and/or 3' recognition sequences and/or selection markers.

In a further aspect, the present invention relates to a host cell, comprising a protein as defined herein, and/or comprising a nucleic acid molecule as defined herein;
wherein, optionally, said host cell is a yeast cell. In one embodiment, the host cell of the present invention expresses said nucleic acid molecule.

In a further aspect, the present invention relates to a method for producing a fatty acid, a fatty alcohol, and/or a fatty aldehyde, comprising contacting the protein, as defined herein, with a substrate selected from acetyl-CoA, malonyl-CoA, NADPH, and combinations thereof; and/or comprising expressing a nucleic acid molecule encoding a protein, said nucleic acid molecule being a nucleic acid molecule as defined herein, in a cell, preferably in a host cell as defined herein.

In one embodiment, said method is a method for producing a C₈ fatty acid, wherein said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
SEQ ID NO: 18 [mFASG113F-'TesA],
SEQ ID NO: 20 [mFASG113F-'TesA4x],
SEQ ID NO: 22 [mFASG113W-'TesA],
SEQ ID NO: 24 [mFASG113W-'TesA4x],
SEQ ID NO: 33 [mFASG113H-'TesA], and
SEQ ID NO: 34 [mFASG113Y-'TesA];

said method is a method for producing a C₁₀ fatty acid, wherein said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
   SEQ ID NO: 10 [mFASG113S-'TesA],
   SEQ ID NO: 17 [mFASG113F-TE_WT],
   SEQ ID NO: 21 [mFASG113W-TE_WT],
   SEQ ID NO: 22 [mFASG113W-'TesA],
   SEQ ID NO: 8 [mFASWT-'TesA4x],
   SEQ ID NO: 12 [mFASG113S-'TesA4x],
   SEQ ID NO: 14 [mFASG113M-'TesA],
   SEQ ID NO: 16 [mFASG113M-'TesA4x],
   SEQ ID NO: 18 [mFASG113F-'TesA],
   SEQ ID NO: 19 [mFASG113F-'TesAL109P],
   SEQ ID NO: 20 [mFASG113F-'TesA4x],
   SEQ ID NO: 31 [mFASG113T-'TesA],
   SEQ ID NO: 32 [mFASG113L-'TesA],
   SEQ ID NO: 33 [mFASG113H-'TesA], and
   SEQ ID NO: 34 [mFASG113Y-'TesA],
   preferably an amino acid sequence of SEQ ID NO: 18 [mFASG113F-'TesA];
said method is a method for producing a C₁₂ fatty acid, wherein said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
   SEQ ID NO: 17 [mFASG113F-TE_WT],
   SEQ ID NO: 18 [mFASG113F-'TesA],
   SEQ ID NO: 21 [mFASG113W-TE_WT],
   SEQ ID NO: 6 [mFASWT-'TesA],
   SEQ ID NO: 8 [mFASWT-'TesA4x],
   SEQ ID NO: 10 [mFASG113S-'TesA],
   SEQ ID NO: 11 [mFASG113S-"TesAL109P],
   SEQ ID NO: 12 [mFASG113S-'TesA4x],
   SEQ ID NO: 14 [mFASG113M-'TesA],
   SEQ ID NO: 15 [mFASG113M-'TesAL109P],
   SEQ ID NO: 16 [mFASG113M-'TesA4x],
   SEQ ID NO: 19 [mFASG113F-'TesAL109P],
   SEQ ID NO: 31 [mFASG113T-'TesA],
   SEQ ID NO: 32 [mFASG113L-'TesA],
   SEQ ID NO: 33 [mFASG113H-'TesA], and
   SEQ ID NO: 34 [mFASG113Y-'TesA];
said method is a method for producing a C₁₄ fatty acid, wherein said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
   SEQ ID NO: 9 [mFASG113S-TE_WT],
   SEQ ID NO: 13 [mFASG113M-TE_WT],
   SEQ ID NO: 17 [mFASG113F-TE_WT],
   SEQ **ID** NO: 21 [mFASG113W-TE_WT],
   SEQ ID NO: 6 [mFASWT-'TesA],
   SEQ ID NO: 8 [mFASWT-'TesA4x],
   SEQ ID NO: 10 [mFASG113S-'TesA],
   SEQ ID NO: 11 [mFASG113S-'TesAL109P],
   SEQ ID NO: 12 [mFASG113S-'TesA4x],
   SEQ ID NO: 14 [mFASG113M-'TesA],
   SEQ ID NO: 15 [mFASG113M-'TesAL109P],
   SEQ ID NO: 16 [mFASG113M-'TesA4x],
   SEQ ID NO: 19 [mFASG113F-'TesAL109P],
   SEQ ID NO: 31 [mFASG113T-'TesA],
   SEQ ID NO: 32 [mFASG113L-'TesA], and
   SEQ ID NO: 33 [mFASG113H-'TesA];
said method is a method for producing a C₁₆ fatty acid, wherein said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
   SEQ ID NO: 9 [mFASG113S-TE_WT],
   SEQ ID NO: 13 [mFASG113M-TE_WT],
   SEQ ID NO: 17 [mFASG113F-TE_WT],
   SEQ ID NO: 21 [mFASG113W-TE_WT],
   SEQ ID NO: 6 [mFASWT-'TesA],
   SEQ ID NO: 8 [mFASWT-'TesA4x],
   SEQ ID NO: 11 [mFASG113S-'TesAL109P], and
   SEQ ID NO: 15 [mFASG113M-'TesAL109P];
said method is a method for producing a C₁₈ fatty acid, wherein said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
   SEQ ID NO: 9 [mFASG113S-TE_WT],
   SEQ ID NO: 13 [mFASG113M-TE_WT],
   SEQ ID NO: 17 [mFASG113F-TE_WT],
   SEQ ID NO: 21 [mFASG113W-TE_WT],
   SEQ ID NO: 7 [mFASWT-'TesAL109P],
   SEQ ID NO: 11 [mFASG113S-'TesAL109P],
   SEQ ID NO: 12 [mFASG113S-'TesA4x], and
   SEQ ID NO: 15 [mFASG113M-'TesAL109P],
   preferably an amino acid sequence of SEQ ID NO: 7 [mFASWT-'TesAL109P]; or
said method is a method for producing a C₈, C₁₀, and/or C₁₂ fatty alcohol and/or C₈, C₁₀, and/or C₁₂ fatty aldehyde, wherein said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
   SEQ ID NO: 28 [mFASG113W-TR2],
   SEQ ID NO: 29 [mFASG113W-TR1], and
   SEQ ID NO: 30 [mFASG113W-TR3]; or
said method is a method for producing a C₁₆, C₁₈, and/or C₂₀ aldehyde, wherein said protein comprises an amino acid sequence of SEQ ID NO: 26 [mFAS-TR1];
wherein, preferably, said method is
a method for producing a C₈ fatty acid, wherein said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
   SEQ ID NO: 20 [mFASG113F-'TesA4x] and
   SEQ ID NO: 24 [mFASG113W-'TesA4x], or
a method for producing a C₈, C₁₀, and/or C₁₂ fatty alcohol and/or C₈, C₁₀, and/or C₁₂ fatty aldehyde, wherein said protein comprises, particularly said one or more polypeptide chains comprise, an amino acid sequence having at least 70%, preferably at least 80%, more preferably at least 90%, even more preferably at least 95% sequence identity, such as at least 98% or 99% sequence identity, to an amino acid sequence selected from
   SEQ ID NO: 28 [mFASG113W-TR2],
   SEQ ID NO: 29 [mFASG113W-TR1], and
   SEQ ID NO: 30 [mFASG113W-TR3].

In one embodiment, said method is said method for producing a C₈ fatty acid, said method for producing a C₁₀ fatty acid, said method for producing a C₁₂ fatty acid, said method for producing a C₁₄ fatty acid, said method for producing a C₁₆ fatty acid, said method for producing a C₁₈ fatty acid, said method for producing a C₈, C₁₀, and/or C₁₂ fatty alcohol, and/or said method for producing a C₈, C₁₀, and/or C₁₂ fatty aldehyde. In one embodiment, said method is said method for producing a C₈ fatty acid, said method for producing a C₁₀ fatty acid, said method for producing a C₁₂ fatty acid, said method for producing a C₁₄ fatty acid, said method for producing a C₁₆ fatty acid, and/or said method for producing a C₁₈ fatty acid. In this aspect, said protein, said nucleic acid, and said host cell are as defined herein.

In a further aspect, the present invention relates to a use of a protein as defined herein, a nucleic acid molecule as defined herein, or a host cell as defined herein, for producing a fatty acid, a fatty alcohol, and/or a fatty aldehyde. For example, said use may comprise performing a method of the invention. In one embodiment, said use comprises producing a C₈ fatty acid, a C₁₀ fatty acid, a C₁₂ fatty acid, a C₁₄ fatty acid, a C₁₆ fatty acid, a C₁₈ fatty acid, a C₈, C₁₀, and/or C₁₂ fatty alcohol, and/or a C₈, C₁₀, and/or C₁₂ fatty aldehyde. In this aspect, said protein, said nucleic acid, and said host cell are as defined herein.

The terms "of the [present] invention", "in accordance with the invention", "according to the invention" and the like, as used herein are intended to refer to all aspects and embodiments of the invention described and/or claimed herein. As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated. The term "about", as used herein in the context of numbers or data, intends to include deviations (±) which usually are to be considered in the respective technical field.

### BRIEF DESCRIPTION OF THE FIGURES

The present invention is now further described by reference to the following figures.

All methods mentioned in the figure descriptions below were carried out as described in detail in the examples.
**Figure 1****. Structure of metazoan FAS, FA synthesis, and design of a generalist mFAS. (a)** Crystallographic structure of porcine mFAS (PDB ID: 2vz8). The homodimeric mFAS adopts an extended X-shaped conformation. Condensing and modifying parts are connected by a short linker and form two lateral reaction clefts. The TE and ACP domains are attached flexibly such that they cannot be traced in electron density. Zoom in one reaction cleft is attached to depict substrate shuttling by ACP. Numbers indicate the sequence of reactions i.e., the path of ACP in shuttling substrates and intermediates to the catalytic domains. **(b)** Cycle of metazoan FA biosynthesis. At defined length, TE releases the acyl chain as free FA. Numbers indicate the sequence of reactions as shown in **a. (c)** Scheme and domain borders of the TE/'TesA swap. A cartoon of mFAS is shown for clarity with one protomer colored. **(d)** Exemplary activity assay of mFAS and the mFAS/'TesA hybrid, monitored by NADPH consumption. The specific activities of wildtype mFAS and mFAS/'TesA were 460 and 458 nmol/min/mg, respectively. **(e)** Product distributions of mFAS and the mFAS/'TesA hybrid. The bars represent the means of three biological replicates and the error bars standard deviations.

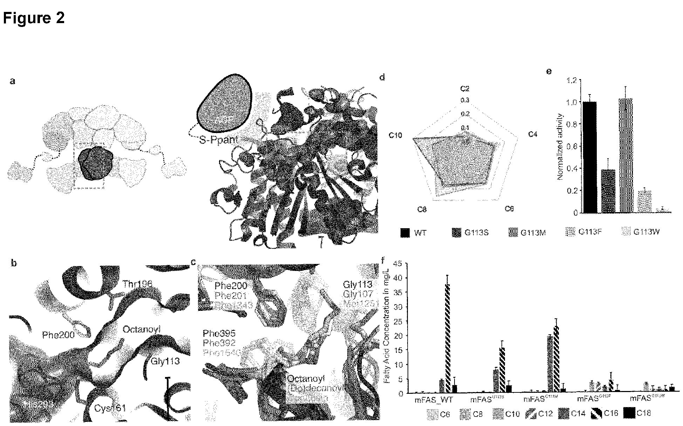

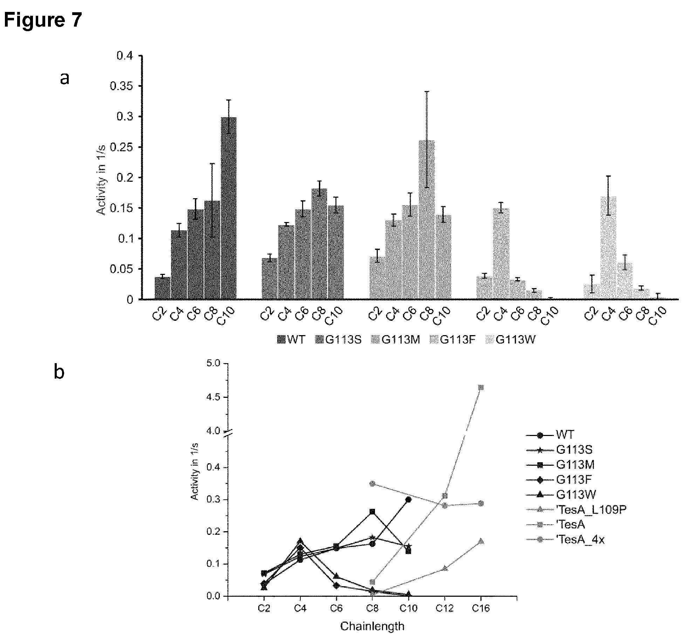

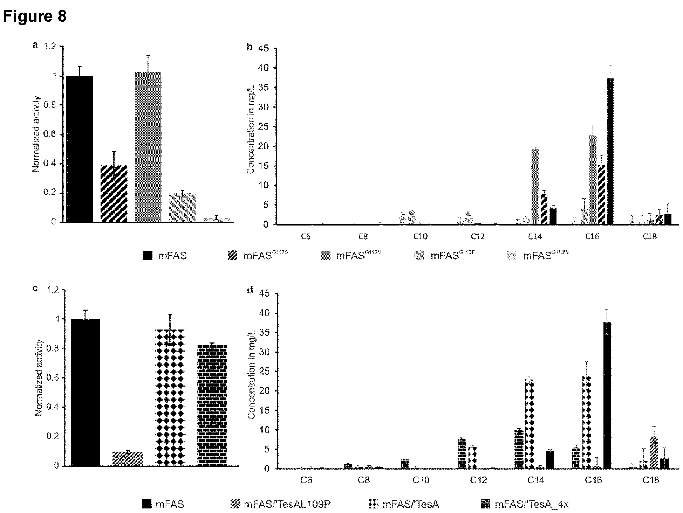

**Figure 2****: Engineering the KS domain. (a)** Cross section of the KS dimer with the binding tunnel represented by its surface in grey. The ACP binding site is indicated. A mFAS model is attached for overview. **(b)** Active site and acyl binding cavity of the KS domain. Key amino acids and the Cys161-bound octanoyl moiety are highlighted. Gly113 is positioned centrally in the binding tunnel near atom C-8 of the substrate. Murine FAS numbering. (c) Superposition with *E. coli* FabB with bound decanoyl and dodecanoyl moieties (PDB IDs: 1f91 (lightcyan) and 1ek4 (darkcyan) and *S. cerevisiae* FAS with bound cerulenin (PDB IDs: 2vkz, lightpink). **(d)** Radar chart of KS elongation rates for substrates of various chain lengths (C2 - C10) of five different KS mutants at position 113. The rates represent three biological replicates and are given in 1/s. Error bars are not given for better visualization and can be found in Figure 7. **(e)** Normalized activity of mFAS_WT and four different KS mutants at position 113 (data collected in biological triplicates with three technical replicates each) as monitored by NADPH consumption. The enzymatic activity of wildtype mFAS was 430 nmol/min/mg. **(f)** Product distributions of mFAS with mutated KS (data collected in biological triplicates with three technical replicates each, error bars refer to standard deviation). FAs were monitored as methyl esters (FAMEs) by gas chromatography (GC) after processing. See also Figure **8b****.**

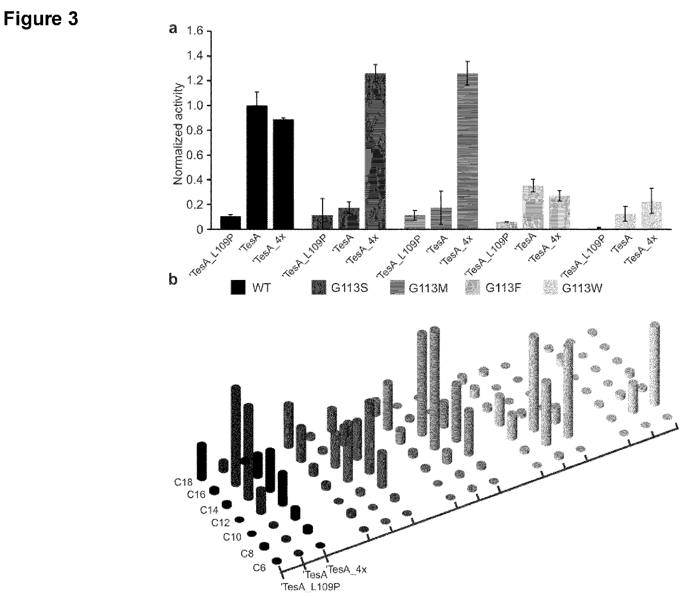

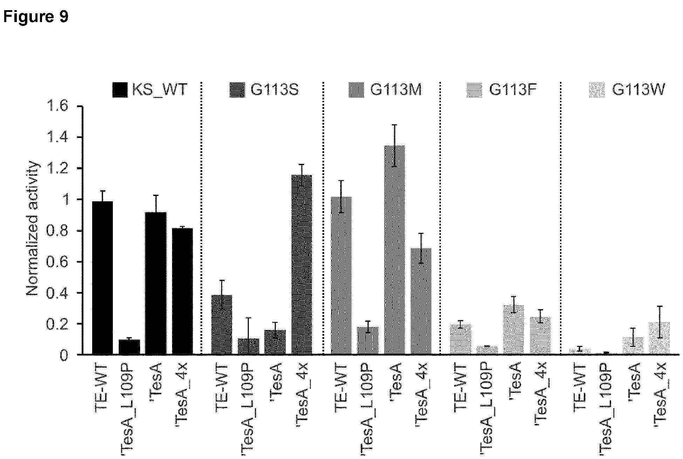

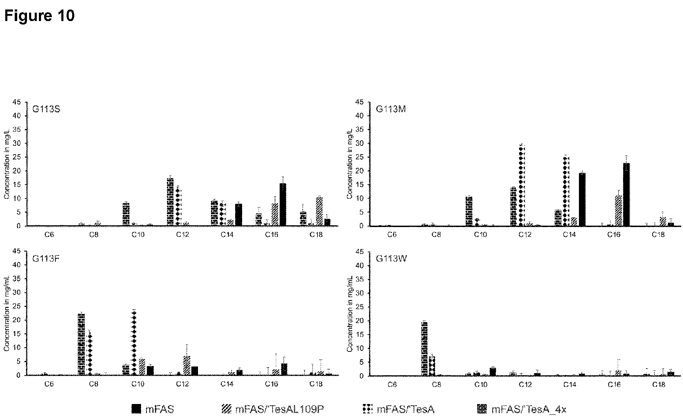

**Figure 3****: In vitro analysis of 12 mFAS/'TesA hybrids engineered in KS and 'TesA. (a)** Normalized activity monitored by NADPH consumption. Data collected in biological triplicates with three technical replicates each, and normalized to mFAS_WT activity. See also Figure 9 **(b)** Product distributions collected in three biological replicates with three technical replicates each. Y-axis and error bars are omitted for better visualization, but can be found in Figure **8a** and **c,** and Figure 10.

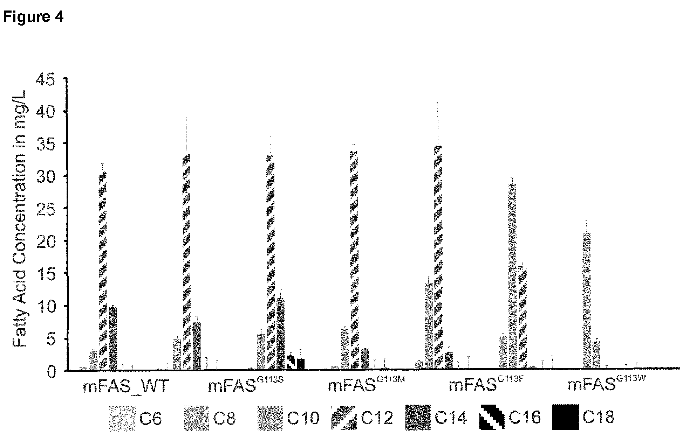

**Figure 4****: In vitro assays of selected mFAS/'TesA hybrids with KS mutations.** FAs were produced in vitro by incubation of the enzyme, Ac-Coa, Mal-CoA and NADPH overnight and measured via gas chromatography. Data collection in three technical replicates each. Error bars represent the standard deviation.

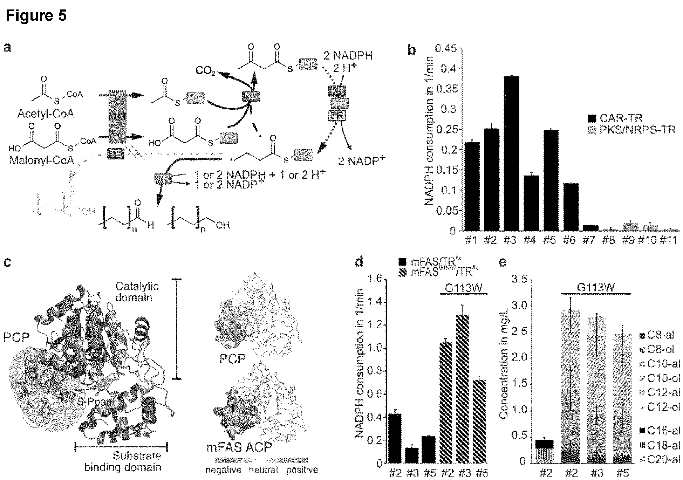

**Figure 5****: Construction of mFAS/TR hybrids for the direct production of short and medium chain length aldehydes/alcohols. (a)** Scheme of aldehyde and alcohol production with mFAS/TR. **(b)** Activity screening of seven TR^{CAR} and four TR^{PKS/NRPS} for the reductive cleavage of the decanoyl group from C10-ACP as decanal and/or decanal. Reductive domains are derived from: *M. marinum* (#1), *M. phlei (#2), M. smegmatis (#3), N. iowensis* (#4), *N. otitidiscaviarum* (#5), *Tsukamurella paurometabola (#6), Neurospora crassa* (#7)*, Stigmatella aurantiaca (#8), M. tuberculosis (#9), M. smegmatis* (#10) and *Methanobrevibacter ruminantium* (#11). Error bars represent the standard deviation of technical triplicates. **(c)** Activity of mFAS/TR hybrids with and without G113W mutation. NADPH consumption (by KR, ER and TR) during fatty alcohol/aldehyde synthesis monitored in three biological replicates. **(d)** *In vitro* production of fatty aldehydes/alcohols with mFAS/TR hybrids. Data from the mFAS/TR hybrids were collected in technical triplicates, data from the hybrids with G113W mutation were collected in three biological and technical replicates each. **(e)** AlphaFold model of PCP-TR^{CAR} from *M. smegmatis* with docked PCP. NADPH is not part of the PCP-TR^{CAR} M. *smegmatis* model, but was superpositioned from the PCP-TR^{CAR} structure from *Segniliparus rugosus* (PDB ID: 5msv). Selected active site residues in stick representation. Vacuum electrostatics indicated for PCP of modeled *M. smegmatis* PCP-TR^{CAR} (right/top), and for mFAS ACP modeled to the PCP atomic coordinates with Modeller (ACP 24% sequence identical to PCP of *M. smegmatis)* (right/bottom).

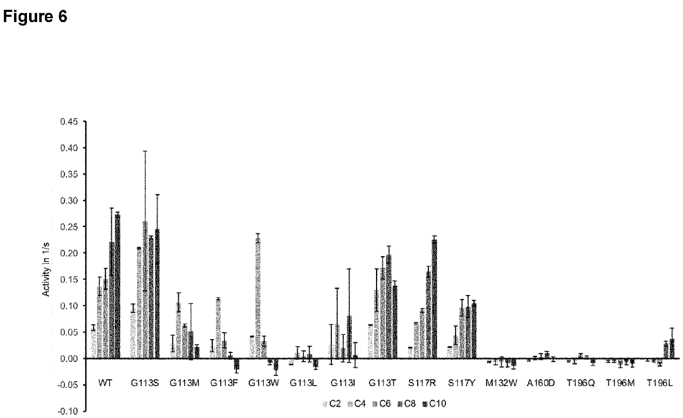

**Figure 6****: Screening of KS mutants.** The initial reaction velocity was determined at vₘₐₓ of 20 µM Mal-ACP in technical duplicated of the wildtype and 16 mutants for acetyl- (C2), butyryl- (C4), hexanoyl- (C6), octanoyl- (C8) and decanoyl- (C10) ACP using the MabA assay. Error bars indicate the propagated error of standard deviations of blank measurement (KS knockout) and positive measurement.
**Figure 7****: KS elongation activity of the WT and four G113 point mutations for chain lengths between C2 and C10. a** The rates represent three biological replicates and are given in 1/s. The error bars reflect the error propagation of blank (knockout) and positive (mutant) measurements standard deviation. b Apparent rates for the substrate octanoyl-ACP of five KS variants (blue) and three 'TesA variants (turquoise) in 1/s.
**Figure 8****: In vitro assays of mFAS KS-mutants and mFAS/'TesA hybrids. a** Normalized activity of five mFAS mutated in KS at position 113 (data collected in biological triplicates with three technical replicates each). NADPH decrease was monitored via its fluorescence (excited 348-320 nm, detected 470-420 nm) over time. Activities of variants were normalized to WT activity. This figure panel is also shown in Figure 2e, and here included for clarity. **b** FAs were produced in vitro by incubation of the enzyme, Ac-Coa, Mal-CoA and NADPH overnight and measured via gas chromatography. This is an accompanying figure to Figure 2f, where FA concentrations sorted by sorted by mFAS variants. **c/d** Normalized activity of different mFAS/'TesA hybrids mutated in 'TesA; including wildtype mFAS (mFAS_WT) as reference. Data collection as described for (a and b) in biological triplicates with three technical replicates each. Figure panels c and d are accompanying figures to Figure 3a and b.
**Figure 9****: Normalized activity of 20 different mFAS constructs.** Data are normalized to WT activity (430 nmol/min/mg protein), and were collected in biological triplicates with three technical replicates each. This is an accompanying figure to Figure 3a.
**Figure 10****: Product distributions of 16 mFAS-hybrids grouped by KS mutants** (data collected in biological triplicates with three technical replicates each). Fatty acids were produced in vitro by incubation of the enzyme, Ac-Coa, Mal-CoA and NADPH overnight and measured via gas chromatography. This is an accompanying figure to Figure 3b.

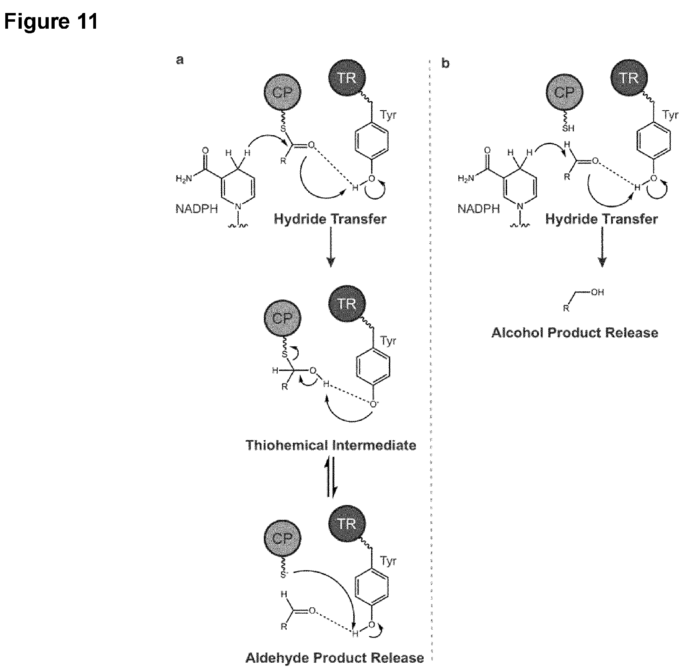

**Figure 11****: Mechanism of TR-mediated reduction and product release. a** Carrier protein (CP) bound substrate is reduced to the corresponding aldehyde using one NADPH as hydride-donor. **b** The formed aldehyde can be reduced to alcohol by a second equivalent of NADPH.

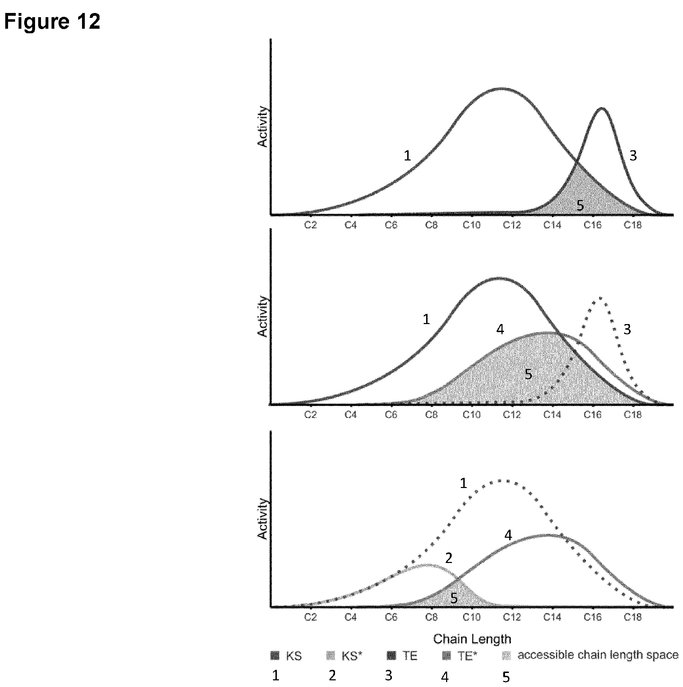

**Figure 12****: Schematic depiction of the kinetic interplay concept for chain length control.** Graphs display estimated activities of KS and TE domains for saturated acyl-ACP across different chain lengths, which will also be referred to as specificity in the following. The intersection reflects the product output spectrum. In the upper panel, KS (dark blue) and TE (brown) reach similar peak activities, although at different chain length; KS at C12 and TE at C16. This kinetic scenario is reflecting the wildtype mFAS with its domains KS and TE. At chain length of C12, the KS-mediated elongation dominates, while the low activity of TE at C12 prevents the TE to intercept chain extension. There is an inverse situation at C16, meaning that the KS activity is decreased at increased TE activity. Here, the TE outcompetes chain extension leading to C16-FAs as main product. The competition for the acyl chain shifts further in favor of the termination reaction by the TE domain for C18 chain length, reflecting the long chain length limit of the wildtype mFAS. When the inventors assume a change in the KS specificity towards shorter chains, possible by KS engineering and represented in the figure by the shift of the KS curve to the left, the intersection diminishes. A slight shift in the chain length output spectrum toward the shorter chain limit of the TE could be expected at the expense of the overall mFAS activity. The mutants mFAS_G113S/F/W reflect such a situation. The panel in the middle displays the situation in which the TE domain (termed TE*) has termination activity at C14 (specificity broadened and shifted towards shorter chains). Here, KS and TE are both able to act on the acyl-ACP and a broadened spectrum is received. This scenario reflects the generalist mFAS/'TesA. As a consequence, the kinetics of the KS mediated elongation influence chain length output. This situation is depicted in the lower figure panel. The chain length output spectra are pushed towards shorter chains, when the KS of the generalist mFAS/'TesA is engineered to decrease elongation activities for chains longer than C6 (indicated by KS*). Since elongation is hampered, the intersection is narrowed and shifted to chains around C8 an C10. This scenario reflects among others variant mFAS^{G113W}/'TesA.

In the following, reference is made to the examples, which are given to illustrate, not to limit the present invention.

### EXAMPLES

### Example 1: Results

The experiments described herein were performed as described in example 2.

*Engineering a generalist variant as production platform.* The inventors performed experiments *in vitro* with full-length mFAS and truncated versions, purified from *Escherichia coli,* enabling to evaluate the effects of scaffold remodeling and mutations in their impact on protein quality, enzyme kinetics and product output.

A mFAS variant from chicken with deleted TE, produced FAs up to C24, indicating that mFAS KS is rather unspecific, and elongates acyl-ACPs with a maximum length of 22 carbons. In contrast, the mFAS TE is specific for C16 due to the hydrophobic binding groove in which the substrate establishes hydrophobic interactions while positioning the thioester bond for productive catalysis. For generating a platform for the production of FAs of various chain length, the inventors replaced the native TE domain with a truncated version of the thioesterase TesA ('TesA) from *E. coli* (Figure 1**c**). 'TesA exhibits broad substrate specificity with a preference for short and medium chain acyl moieties, and has been used for bio-producing SCFAs and MCFAs.

The inventors received a mFAS/'TesA hybrid with rates in fatty acyl extension that remained essentially unchanged compared to wildtype mFAS (Figure 1d). At the same time, the TE/'TesA swap caused a shift in the FA product spectrum towards medium chain lengths (C12-C18). Due to its ideal properties as an engineering platform, the inventors also termed this variant generalist mFAS.

*KS engineering.* The N-terminal KS domain forms a dimer at the center of the condensing region with the binding tunnels extending from the active sites at the center of each protomer toward the dimer interface (Figure **2a****).** The KS performs a two-step reaction comprising the loading of the acyl chain to the active cysteine of KS (transacylation) and elongation of the bound acyl moiety by a C2-unit (decarboxylative Claisen condensation). The specificity of the KS-mediated elongation reaction originates from the transacylation of the acyl chain to the active cysteine (Cys161 in murine mFAS), such that acyl:KS complex structures provide valuable information for engineering. Structural data for murine mFAS with KS-bound octanoyl moiety can directly inform engineering (Figure 2b). Since acyl chain binding is largely conserved across species, the inventors also harnessed related structural information (Figure 2**c**). Finally, the design of KS variants was also guided by homologous KSs from FAS/PKS systems that are known to produce mainly hexanoyl during olivetol biosynthesis.

Without wishing to be bound by any theory, the inventors hypothesized that sterically demanding residues replacing Gly113, Ser117, Met132, Ala160 and Thr196 have an influence on chain length control. Overall, the inventors generated a library of 14 binding tunnel mutants of the KS, which the inventors evaluated in elongation kinetics (Figure 6). Among these variants, Gly113 demonstrated exceptional plasticity to exchanges with bulky sterically demanding residues Ser<Met<Phe<Trp (Figure 2d). Low rates for hexanoyl and octanoyl substrates agree with the steric restrictions imposed by phenylalanine and tryptophan. The turnover rates received for acetyl and butyryl substrates remained essentially unchanged in Gly113 mutants (Figures 7a and b).

KS mutations (G113S/M/F/W) were then integrated into full-length mFAS and subjected to in *vitro* activity and product assays. Except for the G113M variant, the activity of the KS-mutated variants decreased as the size of the amino acid at position 113 increased (Figure 2e). Further, as amino acid size increased, the FA product spectra shifted towards shorter FAs, and FA yields dropped in line with decreased activity (Figures 2**f** and Figure 9).

*'TesA engineering.* In its function to release the acyl chain from the repeating FA cycle, the TE domain competes with KS-mediated elongation (Figure 1b). As a next step, the inventors aimed at regulating the termination reaction by using different 'TesA variants. The inventors harnessed the following variants: i.e., 'TesA_L109P hydrolyzes C8-ACP ten times slower than wildtype, but retains specificity for shorter chains, and 'TesA_M141L/E142D/Y145G/L146K ('TesA_4x) exhibits higher specificity for short chains and a tenfold increased rate for C8-ACP cleavage compared to wildtype (Figure 7**b**). Constructs mFAS/'TesA_4x and mFAS/'TesA_L109P, both containing wildtype KS, had different impact on the FA synthesis. The mFAS/'TesA_4x hybrid operated at similar activity as the mFAS/'TesA (non-mutated 'TesA) and further broadened the spectrum to release C8 to C16-FAs (Figures 3a and b, data highlighted by grey background; see also Figures S9a and b). In contrast, the mFAS/'TesA_L109P exhibited substantially decreased activity in line with low hydrolysis activities of this 'TesA variant. The shift in the product spectrum towards C18-FAs correlates with 'TesA_L109P being ineffective in intercepting the FA cycle driven by fast KS-mediated elongation. Thus, "TesA_L109P can only cleave FAs at chain lengths beyond C16, when KS rates have sufficiently dropped (see also Figure 10).

*Combining KS-engineering and TE-engineering for chain length control.* The inventors then combined four selected KS mutations (G113S, G113M, G113F and G113W) with the three 'TesA variants ('TesA, 'TesA_L109P and 'TesA_4x), yielding 12 mFAS/'TesA hybrids, and recorded catalytic efficiency and product output spectra (Figure 3a and Figure 9). The inventors further introduced four selected KS mutations (G113L, G113T, G113H and G113Y) in the 'TesA variants (Figure 4). While mFAS/'TesA hybrids with aromatic amino acids substituting Gly113 (G113F/W) showed consistently decreased activities, as a result of the throttled KS elongation rates, hybrids with KS mutations G113S and G113M exhibited varying activity depending on the 'TesA variant. In particular, they showed high NADPH consumption rates with 'TesA (G113S-mutated KS) and 'TesA_4x (G113S & G113M). The hybrids mFAS^{G113S}/'TesA_4x and mFAS^{G113M}/'TesA even surpassed rates of the wildtype mFAS, which can be attributed to the high elongation rates of G113S and G113M for short acyl chains that excellently align with the specificity profile of the 'TesA and 'TesA_ 4x. The chain lengths produced are primarily dictated by the KS domain and are further fine-tuned by the matching 'TesA variant, leveraging the kinetic interplay of these two domains (Figures 3b and Figure 10).

*Production of fatty aldehydes and alcohols by a thioester reductase domain.* To extend the mFAS product spectrum beyond FAs, the inventors replaced the TE domain with a TR domain for production of fatty alcohols and/or aldehydes (Figure 5**a**). The inventors focused on two classes of TRs; TRs from carboxylic acid reductases (TRs^{CAR}) and from PKS/NRPS systems (TRs^{PKS}/^{NRPS}). TRs^{PKS/NRPS} catalyze either 2-electron or non-processive (2+2) electron reduction to the aldehyde or alcohol, while TRs^{CAR} strictly perform a 2-electron reduction to the aldehyde. To select a suitable TR to replace the TE^{mFAS} domain, the inventors screened seven TRs^{CAR} and four TRs^{PKS/NRPS}, which represent a broad phylogenetic diversity and are characterized in function and partly in structure.

Stand-alone TRs were tested in their ability to reduce and release saturated C10 acyl chains from mFAS ACP by monitoring NADPH consumption (Figure 5b). The activities of TRs^{PKS/NRPS} were at least one order of magnitude lower than that of TRs^{CAR}, which also naturally process saturated FAs. Further, the inventors observed that the activity of both TR types remains at least two orders of magnitude lower than the overall mFAS activity, indicating that they will impose a kinetic bottleneck when harnessed in mFAS for fatty alcohol/aldehyde production. Structural models of the PCP-TR^{CAR} interaction suggest a direct physical interaction between the domains. An AlphaFold2-modeled *M. smegmatis* PCP:TR complex indicates that the interface is overall little charged, while, in contrast, the mFAS ACP displays a more positively charged surface (Figure 5**e**). Thus, without wishing to be bound by any theory, the inventors hypothesize that suboptimal, non-complementary domain-domain interactions at least partially account for the low efficiency of the reductive release of the acyl chain bound to mFAS.

Based on results from the analysis of stand-alone TR domains, the inventors exchanged the TE^{mFAS} domain with the three best performing TRs, namely the TRs^{CAR} from *Mycobacterium phlei, M. smegmatis* and *Nocardia otitidiscaviarum,* termed in the following TR^{#2}, TR^{#3} and TR^{#5}, respectively. For all constructs, the NADPH consumption rates were low (Figure 5c), and alcohol and aldehyde production could not be detected, except of traces of long chain fatty aldehydes hexadecanal, octadecanal and eicosanal for the hybrid mFAS/TR^{#2} (Figure 5**d**). In seeking to match the kinetics of KS and TR, as the inventors for the kinetic interplay of did with KS and 'TesA in SCFAs and MCFAs production, the inventors eventually decreased the flux in the FA cycle with help of the G113W mutation in KS. In doing so, for all three mFAS/TR hybrids, the activity increased threefold (Figure 5**c**), and product amounts for hybrid mFAS^{G113W}/TR^{#2} increased up to eightfold (Figure 5**d**).

### Example 2: Materials and Methods

### Plasmids

Plasmids were constructed using either In-Fusion Cloning (Takara), Gibson Assembly, SOE-PCR and restriction enzyme cloning. The DNA encoding for the TR domains was amplified from their genomic DNA or a plasmid from another group and introduced into a pET-22b (+) vector.

### Protein Expression and Purification

### Heterologous Expression of mFAS Constructs

Plasmids containing full length mFAS constructs and mFAS hybrids were transformed into electrically competent *E. coli* BAP1 cells and plasmids containing KS-MAT° were transformed into chemically competent *E. coli* BL21 gold (DE3). After transformation cells were plated on LB-Agar plates (100 µg/mL ampicillin (amp) and 1% (w/v) glucose). 3-6 colonies were picked and cells were grown overnight (ON) at 37 °C in 20 mL LB medium (100 µg/mL amp and 1% (w/v) glucose). Pre-cultures were used to inoculate 1 L TB medium (100 µg/mL amp). Cultures were grown at 37 °C and 150 rpm until they reached the desired optical density (OD₆₀₀) of 0.6-0.8. After cooling at 4 °C, cultures were induced with 250 µM IPTG, and grown for additional 16 h at 20 °C and 150 rpm. Cells were harvested by centrifugation (5,000 rcf for 15 min). After the supernatant was discarded, the remaining cell pellet was resuspended in 30 mL lysis buffer (200 mM KCl, 30 mM imidazole, 50 mM potassium phosphate, 10% (v/v) glycerol, 1mM EDTA, pH 7.0), a small amount DNaseI (Sigma Aldrich) was added. A French Pressure Cell Press (Amico) was used to disrupt the cells. The resulting suspension was centrifuged using the JA 25.500 rotor at 40000 rcf for 1 h. The supernatant was mixed with 1 M MgCl₂ to a final concentration of 2 mM and then transferred to Ni-NTA-columns and washed with 5 column volumes (CV) his-wash buffer (lysis buffer without EDTA). Bound protein was eluted with 5 mL elution buffer (200 mM KCl, 300 mM imidazole, 50 mM potassium phosphate, 10% (v/v) glycerol, pH 7.0). The eluted protein was then transferred to a Strep-Tactin column. After being washed with 5 CV strep-wash buffer (250 mM potassium phosphate, 1 mM EDTA, 10% (v/v) glycerol, pH 7.0), bound protein was eluted with 5 mL strep-elution buffer (strep-wash buffer + 40 mM biotin). Afterwards proteins were concentrated to 5-10 mg/mL, frozen in liquid nitrogen and stored at -80 °C. Next, proteins were thawed at 37 °C for 60 min and further purified by size-exclusion chromatography (SEC). A Superdex 200 GL 10/300 column and strep-wash buffer were used. Fractions with dimeric protein were pooled and subsequently concentrated to 2-5 mg/mL. Aliquots were frozen in liquid nitrogen and stored at -80 °C.

### Heterologous Expression of mFAS ACP and TR domains

Plasmids containing mFAS-ACP and the TR-Domains were transformed into chemically competent *E. coli* BL21 gold (DE3). They were expressed as the mFAS constructs but no Strep-Tactin purification was performed. SEC was done with a Superdex 200 GL 10/300 column and his-wash buffer were used. The protein fractions containing the correct size were pooled and concentrated to 1-10 mg/mL for the TR domains and 40-60 mg/mL for the mFAS-ACP. Aliquots were frozen in liquid nitrogen and stored at -80 °C.

### Chemical Synthesis: Synthesis of decanoyl-CoA ester

The synthesis was performed under argon-atmosphere. 202 mg decanoic acid (1.17 mmol, 6 eq) was dissolved in 6 mL abs. THF and cooled to 0 °C. 90 µL ethyl chloroformate (1.16 mmol, 5.9 eq) and 160 µL triethylamine (TEA) (1.15 mmol, 5.9 eq) were added to the solution and stirred for 45 min at 0 °C. The reaction mixture was transferred to a 2 mL Eppendorf-tube and centrifuged for 5 min at 20000 x g. The supernatant was transferred to a solution of 150 mg CoA (0.195 mmol, **1** eq) in 6 mL 0.1 M NaHCO₃ and stirred for 1 h at RT. The solution was divided into two parts and each fraction was poured into 25 mL -20 °C cold acetone. The mixtures were centrifuged for 5 min at 10000 x g and the supernatant was discarded. The crude product was dried under reduced pressure and stored at -20 °C until further purification. Isolation of the product was performed using a reverse phase (RP) liquid chromatography (LC) column with C18-RP silica gel (part. size 48-65) from Thermo Scientific and MeOH/H2O 1:1 as eluent. The collected fractions were combined and the organic solvent removed under reduced pressure. The yield was determined photometrically via the absorption at 260 nm and the purity of the product was determined by high performance liquid chromatography (HPLC) analysis. The sample was injected to the AcclaimTM Polar Advantage II LC-column from Thermo Scientific and eluted with a buffer (200 mM ammonium acetate, pH 6)/ACN gradient (5-60% in 23 min). **Yield:** 45% as white solid.

### Biochemical Synthesis: Synthesis of C10-ACP

C10-ACP was biosynthesized using the phosphopantetheine transferase SFP to load an acyl-Ppant from acyl-CoA onto apo-ACP.

SFP, C10-CoA and apo-ACP were mixed in 5 mL buffer (50 mM Tris, 10 mM MgCl₂, pH 7.5) to final concentrations of: SFP (15 µM, 0.05 eq), C10-CoA (1500 µM, 5 eq) and apo-ACP (300 µM, 1 eq). The reaction mixture was incubated for 10 min at 37 °C and 300 rpm and the reaction progress was checked via HPLC analysis. Therefore, 5 µL sample were mixed with 25 µL 0.1% TFA solution and injected to the Discovery^{®} BIO Wide Pore C5 LC-column from Supelco and eluted with a H₂O + 0.1%TFA/ACN + 0.1%TFA gradient (20-98 % in 16 min). The reaction mixture was poured onto a Strep Tactin XT column, washed with 3 CV Strep-Wash buffer and eluted with 2.5 CV Strep-Elution buffer. The elution fraction was rebuffered to ACP-buffer (50 mM potassium phosphate, 200 mM KCl, 1 mM EDTA, 10% (v/v) glycerol, pH 7.0) and concentrated using Amicon^{®} Ultra Centrifugal filters from Merck with a 10 kDa cut-off. The purification procedure was monitored via HPLC analysis.

### Enzymatic Assays

### MabA Assay

Condensation activity of the KS domain was monitored indirectly with an enzyme coupled assay using a reductase MabA from *M. tuberculosis.* All solutions were prepared in an assay buffer (50 mM sodium phosphate, 10% glycerol, pH 7.0) as 8x stock or 2x stock (Mal-ACP) and pipetted immediately before monitoring. The components were added to a final concentration of 500 nM enzyme, 20 µM Mal-ACP, 100 µM Acyl-ACP, 50 µM NADPH and 5 µM MabA. The NADPH fluorescence (excited 348-320 nm, detected 476-420 nm) was monitored during the reaction at 25 °C.

### mFAS Activity Assays

Measurements of the mFAS based constructs were done using a *CLARIOstar*^{®} *Plus Microplate Reader* and 386-well-plates (Greiner). NADPH fluorescence was measured (excited 348-320 nm, detected 470-420 nm) at 25°C. The assay was performed in 20 µL assay volume. All solutions were prepared in an assay buffer (50 mM potassium phosphate, 10% glycerol (v/v), 5% PEG 400 (v/v), 0.03 mg/mL BSA, 1 mM DTT, pH 7,0) as 4x stock and pipetted immediately before monitoring. The components were added to a final concentration of 100 µM Acetyl-CoA, 100 µM Malonyl-CoA, 50 µM NADPH and 20-80 nM enzyme (WT and chain length constructs) or 400-800 nM enzyme (TR-hybrids). Prior to the measurements each protein was thawed for 5 min at 37°C and stored on ice until the measurement. Blank measurements were conducted without acetyl- and malonyl-CoA.

### TR Activity Screening

Measurements of the standalone TR domains were done using a *CLARIOstar*^{®} *Plus Microplate Reader* and 386-well-plates (Greiner). NADPH fluorescence was measured (excited 348-320 nm, detected 470-420 nm) at 25°C. The assay was performed in 21 µL assay volume. All solutions were prepared in an assay buffer (50 mM potassium phosphate, 10 mM MgCl₂, 10% glycerol (v/v), pH 7.0) as 3x stock and pipetted immediately before monitoring. The components were added to a final concentration of 10 µM NADPH, 460 µM C10-mFAS ACP and 1 µM TR. Prior to the measurements each protein was thawed for 5 min at 37°C and stored on ice until the measurement. Blank measurements were conducted with apo-mFAS ACP instead of C10-mFAS ACP.

### In vitro Fatty Acid Production Assay

The *in vitro* production of fatty acids was performed similar to the mFAS activity assays. The assay was performed in 500 µL assay volume. All solutions were prepared in an assay buffer (50 mM potassium phosphate, 10% glycerol (v/v), 5% PEG 400 (v/v), 0.03 mg/mL BSA, 1 mM DTT, pH 7.0) as 4x stock and added to final concentrations of 500 µM Acetyl-CoA, 500 µM Malonyl-CoA, 500 µM NADPH and 40 nM enzyme concentration. The assay solution was then incubated ON at RT. After incubation 5 µL of heptanoic acid (2 g/L in methanol/CHCl₃ 3:1) was added as internal standard for the GC measurements.

### Fatty Acid Extraction and Derivatization

To increase the vapor pressure and decrease the boiling point of the FAs produced in vitro, the FAs were converted to fatty acid methyl esters (FAMEs). Therefore, the 500 µL test solution was acidified with 50 µL of 8% HCl in methanol to protonate the FAs and make them less polar. Next, 125 µL MeOH/CHCl3 (1:1) was added and the solution was vortexed to extract the fatty acids to the non-polar phase. The mixture was then centrifuged at 3000 x g for 10 min to separate the phases. The lower non-polar phase was transferred to a fresh Eppendorf tube and the solvent was evaporated under reduced pressure. 20 µL toluene was added and transferred to Duran glass tubes containing 200 µL solution of 8% HCl in MeOH and 500 µL MeOH. The tubes were sealed tightly and shaken to mix the solutions. The reaction mixture was then heated to 100°C for 3 hours. The reaction was then cooled on ice for 10 min and 100 µL of hexane and 300 µL of H2O were added. The mixture was then vortexed and returned to ice to allow the phases to separate. 70 µL of the hexane phase was transferred to a GC vial with micro inlet.

### In vitro Fatty Aldehyde/Alcohol Production Assay

The *in vitro* production of fatty acids was performed similar to the mFAS activity assays. The assay was performed in 500 µL assay volume. All solutions were prepared in an assay buffer (50 mM potassium phosphate, 10% glycerol (v/v), 5% PEG 400 (v/v), 0.03 mg/mL BSA, 1 mM DTT, pH 7.0) as 4x stock and added to final concentrations of 300 µM Acetyl-CoA, 300 µM Malonyl-CoA, 500 µM NADPH and 1 µM enzyme concentration. The assay solution was overlaid with 100 µL hexane and then incubated ON at RT. After incubation 10 µL of heptanoic acid (0.4 g/L in hexane) was added as internal standard for the GC measurements.

### Fatty Aldehyde/Alcohol Extraction

Extraction was done by adding another 50 µL hexane and mixing. The mixture was centrifuged 10 min at 5000 x g for phase separation. Finally, 50 µL of the organic phase was transferred to a GC vial with micro inlet.

### Gas Chromatography Measurements

The measurements of the FAMEs have been conducted with two different devices: a Perkin Elmer Clarus 400 gas chromatograph equipped with an Elite 5MS capillary column (30 m × 0.25 mm, film thickness: 0.25 µm) and a flame ionization detector; And an Agilent 7890B gas chromatograph equipped with a HP-5ms Ultra Inert capillary column (30 m × 0.25 mm, film thickness: 0.25 µm) and an Agilent 5977B MSD. The injector temperature was 200 °C and the detector temperature was 250 °C. The temperature program used was: Heating to 50 °C for 5 min; increase of 10 °C/min until 120 °C was reached, then hold for 5 min; increase of 15 °C/min until 220 °C was reached and then hold for 10 min.

The measurements of FA aldehydes/alcohols have been conducted with an Agilent 7890B gas chromatograph equipped with an HP-5ms Ultra Inert capillary column (30 m × 0.25 mm, film thickness: 0.25 µm) and an Agilent 5977B MSD. The temperature program used was: Heating to 50°C for 7 min; increase of 15 °C/min until 220 °C was reached, then hold for 5 min; increase of 20 °C/min until 280 °C was reached and then hold for 5 min.

### GC Data Analysis

The peaks at the previously obtained retention times of each FAME were integrated using the program *totalchrom* for the Perkin Elmer data, *OpenChrom* was used for the Agilent data. To take losses during extraction and derivatization into account the signals was normalized to the signal of the internal standard. The final concentrations of the FAMEs were calculated using following formula: $csample = \frac{Asample}{msample} ⋅ \left(\left(\frac{mstandard}{Astandard}⋅Cstandard\right)⋅1000\right)$

With Aₛₐₘₚₗₑ being the integrated peak area of the respective sample peak, A_{standard} being the integrated peak area of the standard peak, mₛₐₘₚₗₑ being the slope of the respective sample calibration curve, m_{standard} being the slope of the standards calibration curve and cₛₐₘₚₗₑ and c_{standard} the corresponding concentrations.

### Example 3: Conclusion

In metazoan de novo FA biosynthesis, the chain length of products is primarily regulated by the substrate-specific TE^{mFAS} domain. In addition, the KS domain supports chain length regulation by throttling the turnover rate of the FA cycle as acyl chains approach C16. In this work, the inventors aimed to adapt native chain regulation to establish mFAS-based biosynthesis of FAs of different chain lengths. To do so, the inventors followed a two-step approach: First, the inventors installed a new and more promiscuous TE domain, and second, the inventors re-adjusted the kinetics of FA cycle to the new requirements of SCFA and MCFA production.

To this end, the inventors employed 'TesA as the terminating domain due to its reported broader substrate specificity, resulting in a generalist mFAS, which has lost its strict chain length control. The viability of this approach is reflected by the expansion of the product spectrum to C12- to C18-FAs (Figure **1e**). This generalist mFAS was subsequently modulated in the kinetics of the KS and the 'TesA domain to create specialized variants for SCFAs and MCFAs (Figure 3b). With amino acid Gly113, the inventors identified a hotspot for modulating the chain-length-dependent efficiency of KS in acyl chain elongation. Single amino acid exchanges in Gly113 can shift product spectra from C8-FAs (G113W) to C8/C10-FAs (G113F) and C12/C14-FAs (G113M). The efficiency of the mFAS/'TesA variants is high. Remarkably, mFAS/'TesA hybrids producing MCFAs showed partly even higher FA cycle turnover rates than the wildtype mFAS, and the SCFAs producing hybrids that rely on throttled turnover of the FA cycle were significantly attenuated in activity. For hybrid mFAS^{G113W}/'TesA_4x, producing 95% C8-FA, activity dropped for to about 20% of WT activity (corresponding to eight molecules per minute per mFAS dimer; Figure 9).

The inventors further extended the approach to the production of short and medium chain length fatty aldehydes and alcohols by designing mFAS/TR hybrids via a similar strategy as for the design of mFAS/'TesA hybrids. The inventors were able to create active constructs (Figure 5e).

### Example 4: Further findings

The inventors have found that KS is very fast for substrates between a chain length of C8 to C14. Therefore, variants that produce fatty acids in the chain length range of fast kinetics can be more efficient than the native FAS. The inventors have found that variants characterized by KS mutation and/or the presence of 'TesA may be such fast variants. Since KS is slow for short substrates, C8-producing variants are inevitably less fast/efficient than the native FAS. However, these variants are still valuable, since the products of such variants are very valuable products. In summary, all the variants (particularly all slow and fast variants) described herein are relevant because they all produce specific products.

The features of the present invention disclosed in the specification, the claims, and/or in the accompanying figures may, both separately and in any combination thereof, be material for realizing the invention in various forms thereof.

## Claims

1. A protein, preferably a fatty acid synthase, comprising one or more polypeptide chains, wherein said one or more polypeptide chains comprise
(i) an amino acid sequence encoding a metazoan ketoacyl synthase (KS) domain, preferably an amino acid sequence encoding a murine KS domain; and/or
(ii) an amino acid sequence encoding a bacterial thioesterase ('TesA) domain, preferably an amino acid sequence encoding a thioesterase domain of E. coli; or
an amino acid sequence encoding a thioreductase (TR) domain, preferably an amino acid sequence encoding a bacterial TR domain.

2. The protein according to claim 1, wherein said amino acid sequence encoding a metazoan KS domain has at least 70%, preferably at least 80%, more preferably at least 90%, even more preferably at least 95% sequence identity to the amino acid sequence of SEQ ID NO: 1;
wherein said amino acid sequence encoding a bacterial 'TesA domain has at least 70%, preferably at least 80%, more preferably at least 90%, even more preferably at least 95% sequence identity to the amino acid sequence of SEQ ID NO: 2; and/or
wherein said amino acid sequence encoding a TR domain has at least 70%, preferably at least 80%, more preferably at least 90%, even more preferably at least 95% sequence identity to the amino acid sequence of SEQ ID NO: 3, 4, or 5.

3. The protein according to claim 1 or 2, wherein the amino acid sequence encoding a metazoan KS domain has at least 70%, preferably at least 80%, more preferably at least 90%, even more preferably at least 95% sequence identity to the amino acid sequence of SEQ ID NO: 1 and comprises an amino acid substitution in the KS domain at a position corresponding to G113, S117, or T196 of the amino acid sequence of SEQ ID NO: 1;
wherein, preferably, said amino acid substitution is selected from G113S, G113M, G113F, G113W, G113L, G113I, G113T, G113H, G113Y, S117R, S117Y, and T196L; wherein, more preferably, said amino acid substitution is selected from G113S, G113M, G113F, G113W, G113L, G113T, G113H, and G113Y;
wherein, even more preferably, said amino acid substitution is selected from G113S, G113M, G113F, and G113W.

4. The protein according to any one of the foregoing claims, wherein the amino acid sequence encoding a 'TesA domain has at least 70%, preferably at least 80%, more preferably at least 90%, even more preferably at least 95% sequence identity to the amino acid sequence of SEQ ID NO: 2 and comprises one or more amino acid substitutions in the 'TesA domain at a position corresponding to L109, M141, E142, Y145, and/or L146 of the amino acid sequence of SEQ ID NO: 2;
wherein, preferably, said one or more amino acid substitutions are selected from L109P, M141L, E142D, Y145G, L146K, and combinations thereof;
wherein, more preferably, the amino acid sequence encoding a 'TesA domain comprises amino acid substitution L109P or comprises amino acid substitutions M141L, E142D, Y145G, and L146K.

5. The protein according to any one of the foregoing claims, wherein the TR domain is a TR domain of a *Mycobacterium* sp., a *Nocardia* sp., a *Tsukamurella* sp., a *Neurospora* sp., a *Stigmatella* sp., or *Methanobrevibacter* sp.; optionally a TR domain of a carboxylic acid reductase (CAR) or of a hybrid polyketide synthase non-ribosomal peptide synthetase (PKS-NRPS), preferably a TR domain of a CAR;
wherein, preferably, the TR domain is a TR domain of *Mycobacterium marinum, Mycobacterium phlei, Mycobacterium smegmatis, Mycobacterium tuberculosis, Nocardia iowensis, Nocardia otitidiscaviarum, Tsukamurella paurometabola, Neurospora crassa, Stigmatella aurantiaca,* or *Methanobrevibacter ruminantium;*
wherein, more preferably, the TR domain is a TR domain of *Mycobacterium phlei, Mycobacterium smegmatis,* or *Nocardia otitidiscaviarum,* optionally a TR domain of a CAR of *Mycobacterium phlei, Mycobacterium smegmatis,* or *Nocardia otitidiscaviarum.*

6. The protein according to any one of the foregoing claims, wherein said one or more polypeptide chains comprise
(i) said amino acid sequence encoding a metazoan ketoacyl synthase (KS) domain, preferably said amino acid sequence encoding a murine KS domain; and
(ii) said amino acid sequence encoding a bacterial thioesterase ('TesA) domain, preferably said amino acid sequence encoding a thioesterase domain of E. coli; or
said amino acid sequence encoding a thioreductase (TR) domain, preferably said amino acid sequence encoding a bacterial TR domain;
or wherein said one or more polypeptide chains comprise
(i) said amino acid sequence encoding a metazoan ketoacyl synthase (KS) domain; wherein the amino acid sequence encoding a metazoan KS domain has at least 70%, preferably at least 80%, more preferably at least 90%, even more preferably at least 95% sequence identity to the amino acid sequence of SEQ ID NO: 1 and comprises an amino acid substitution in the KS domain at a position corresponding to G113, S117, or T196 of the amino acid sequence of SEQ ID NO: 1;
wherein, preferably, said amino acid substitution is selected from G113S, G113M, G113F, G113W, G113L, G113I, G113T, G113H, G113Y, S117R, S117Y, and T196L;
wherein, more preferably, said amino acid substitution is selected from G113S, G113M, G113F, G113W, G113L, G113T, G113H, and G113Y; wherein, even more preferably, said amino acid substitution is selected from G113S, G113M, G113F, and G113W; and
(ii) an amino acid sequence encoding a thioesterase domain, preferably a metazoan thioesterase domain, more preferably a thioesterase domain having at least 70%, preferably at least 80%, more preferably at least 90%, even more preferably at least 95% sequence identity to the amino acid sequence of SEQ ID NO: 35.

7. The protein according to any one of the foregoing claims, wherein the one or more polypeptide chains comprise
- an amino acid sequence encoding a metazoan KS domain having at least 70%, preferably at least 80%, more preferably at least 90%, even more preferably at least 95% sequence identity to the amino acid sequence of SEQ ID NO: 1; and
an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1; and an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity to the amino acid sequence of SEQ ID NO: 1; and an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113S; and
an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113S; and
an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113S; and
an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113M; and
an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113M; and
an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113M; and
an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113F; and
an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113F; and
an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113F; and
an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113W; and
an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113W; and
an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitution L109P; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113W; and
an amino acid sequence encoding a 'TesA domain having at least 70%, preferably at least 80%, more preferably at least 90%, even more preferably at least 95% sequence identity to the amino acid sequence of SEQ ID NO: 2 and comprising the amino acid substitutions M141L, E142D, Y145G, and L146K; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113T; and
an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113L; and
an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113H; and
an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113Y; and
an amino acid sequence encoding a 'TesA domain, preferably having at least 70%, preferably at least 80%, more preferably at least 90%, even more preferably at least 95% sequence identity to the amino acid sequence of SEQ ID NO: 2; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113S; and
an amino acid sequence encoding a TR domain having at least 70%, preferably at least 80%, more preferably at least 90%, even more preferably at least 95% sequence identity to the amino acid sequence of SEQ ID NO: 3, 4, or 5; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113M; and
an amino acid sequence encoding a TR domain having at least 70%, preferably at least 80%, more preferably at least 90%, even more preferably at least 95% sequence identity to the amino acid sequence of SEQ ID NO: 3, 4, or 5; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113F; and
an amino acid sequence encoding a TR domain having at least 70%, preferably at least 80%, more preferably at least 90%, even more preferably at least 95% sequence identity to the amino acid sequence of SEQ ID NO: 3, 4, or 5; or
- an amino acid sequence encoding a metazoan KS domain having at least 70%, preferably at least 80%, more preferably at least 90%, even more preferably at least 95% sequence identity to the amino acid sequence of SEQ ID NO: 1 and comprising the amino acid substitution G113W; and
an amino acid sequence encoding a TR domain having at least 70%, preferably at least 80%, more preferably at least 90%, even more preferably at least 95% sequence identity to the amino acid sequence of SEQ ID NO: 3, 4, or 5.

8. The protein according to any one of the foregoing claims, wherein said protein comprises an amino acid sequence selected from
SEQ ID NO: 6 [mFASWT-'TesA],
SEQ ID NO: 7 [mFASWT-'TesAL109P],
SEQ ID NO: 8 [mFASWT-'TesA4x],
SEQ ID NO: 9 [mFASG113S-TE_WT],
SEQ ID NO: 10 [mFASG113S-'TesA],
SEQ ID NO: 11 [mFASG113S-'TesAL109P],
SEQ ID NO: 12 [mFASG113S-'TesA4x],
SEQ ID NO: 13 [mFASG113M-TE_WT],
SEQ ID NO: 14 [mFASG113M-'TesA],
SEQ ID NO: 15 [mFASG113M-'TesAL109P],
SEQ ID NO: 16 [mFASG113M-'TesA4x],
SEQ ID NO: 17 [mFASG113F-TE_WT],
SEQ ID NO: 18 [mFASG113F-'TesA],
SEQ ID NO: 19 [mFASG113F-'TesAL109P],
SEQ ID NO: 20 [mFASG113F-'TesA4x],
SEQ ID NO: 21 [mFASG113W-TE_WT],
SEQ ID NO: 22 [mFASG113W-'TesA],
SEQ ID NO: 23 [mFASG113W-'TesAL109P],
SEQ ID NO: 24 [mFASG113W-'TesA4x],
SEQ ID NO: 31 [mFASG113T-'TesA],
SEQ ID NO: 32 [mFASG113L-'TesA],
SEQ ID NO: 33 [mFASG113H-'TesA], and
SEQ ID NO: 34 [mFASG113Y-'TesA];
wherein, preferably, said protein comprises an amino acid sequence selected from
SEQ ID NO: 10 [mFASG113S-'TesA],
SEQ ID NO: 11 [mFASG113S-'TesAL109P],
SEQ ID NO: 12 [mFASG113S-'TesA4x],
SEQ ID NO: 13 [mFASG113M-TE_WT],
SEQ ID NO: 14 [mFASG113M-'TesA],
SEQ ID NO: 15 [mFASG113M-'TesAL109P],
SEQ ID NO: 16 [mFASG113M-'TesA4x],
SEQ ID NO: 18 [mFASG113F-'TesA],
SEQ ID NO: 19 [mFASG113F-'TesAL109P],
SEQ ID NO: 20 [mFASG113F-'TesA4x],
SEQ ID NO: 22 [mFASG113W-'TesA],
SEQ ID NO: 23 [mFASG113W-'TesAL109P],
SEQ ID NO: 24 [mFASG113W-'TesA4x],
SEQ ID NO: 31 [mFASG113T-'TesA],
SEQ ID NO: 32 [mFASG113L-'TesA],
SEQ ID NO: 33 [mFASG113H-'TesA], and
SEQ ID NO: 34 [mFASG113Y-'TesA];
wherein, optionally, said protein comprises an amino acid sequence selected from
SEQ ID NO: 10 [mFASG113S-'TesA],
SEQ ID NO: 12 [mFASG113S-'TesA4x],
SEQ ID NO: 14 [mFASG113M-'TesA], and
SEQ ID NO: 16 [mFASG113M-'TesA4x],
such as an amino acid sequence selected from
SEQ ID NO: 12 [mFASG113S-'TesA4x] and
SEQ ID NO: 14 [mFASG113M-'TesA].

9. The protein according to any one of claims 1-7, wherein said protein comprises an amino acid sequence selected from
SEQ ID NO: 25 [mFAS-TR2],
SEQ ID NO: 26 [mFAS-TR1],
SEQ ID NO: 27 [mFAS-TR3],
SEQ ID NO: 28 [mFASG113W-TR2],
SEQ ID NO: 29 [mFASG113W-TR1], and
SEQ ID NO: 30 [mFASG113W-TR3];
wherein, preferably, said protein comprises an amino acid sequence selected from
SEQ ID NO: 25 [mFAS-TR2]
SEQ ID NO: 28 [mFASG113W-TR2],
SEQ ID NO: 29 [mFASG113W-TR1], and
SEQ ID NO: 30 [mFASG113W-TR3];
wherein, more preferably, said protein comprises an amino acid sequence selected from
SEQ ID NO: 28 [mFASG113W-TR2],
SEQ ID NO: 29 [mFASG113W-TR1], and
SEQ ID NO: 30 [mFASG113W-TR3];
wherein, even more preferably, said protein comprises an amino acid sequence of
SEQ ID NO: 28 [mFASG113W-TR2].

10. The protein according to any one of the foregoing claims, wherein the protein does not comprise a thioesterase (TE) domain.

11. A nucleic acid molecule encoding a protein according to any one of the foregoing claims.

12. A host cell, comprising a protein according to any one of claims 1-10 and/or a nucleic acid molecule according to claim 11;
wherein, optionally, said host cell is a yeast cell.

13. A method for producing a fatty acid, a fatty alcohol, and/or a fatty aldehyde, comprising contacting the protein according to any one of claims 1-10 with a substrate selected from acetyl-CoA, malonyl-CoA, NADPH, and combinations thereof; and/or comprising expressing a nucleic acid molecule encoding a protein, said nucleic acid molecule being a nucleic acid molecule according to claim 11, in a cell, preferably in a host cell according to claim 12.

14. The method according to claim 13,
wherein said method is a method for producing a C₈ fatty acid, wherein said protein comprises an amino acid sequence selected from
SEQ ID NO: 18 [mFASG113F-'TesA],
SEQ ID NO: 20 [mFASG113F-'TesA4x],
SEQ ID NO: 22 [mFASG113W-'TesA],
SEQ ID NO: 24 [mFASG113W-'TesA4x],
SEQ ID NO: 33 [mFASG113H-'TesA], and
SEQ ID NO: 34 [mFASG113Y-'TesA];
wherein said method is a method for producing a C₁₀ fatty acid, wherein said protein comprises an amino acid sequence selected from
SEQ ID NO: 10 [mFASG113S-'TesA],
SEQ ID NO: 17 [mFASG113F-TE_WT],
SEQ ID NO: 21 [mFASG113W-TE_WT],
SEQ ID NO: 22 [mFASG113W-'TesA],
SEQ ID NO: 8 [mFASWT-'TesA4x],
SEQ ID NO: 12 [mFASG113S-'TesA4x],
SEQ ID NO: 14 [mFASG113M-'TesA],
SEQ ID NO: 16 [mFASG113M-'TesA4x],
SEQ ID NO: 18 [mFASG113F-'TesA],
SEQ ID NO: 19 [mFASG113F-'TesAL109P],
SEQ ID NO: 20 [mFASG113F-'TesA4x],
SEQ ID NO: 31 [mFASG113T-'TesA],
SEQ ID NO: 32 [mFASG113L-'TesA],
SEQ ID NO: 33 [mFASG113H-'TesA], and
SEQ ID NO: 34 [mFASG113Y-'TesA], preferably an amino acid sequence of SEQ ID NO: 18 [mFASG113F-'TesA];
wherein said method is a method for producing a C₁₂ fatty acid, wherein said protein comprises an amino acid sequence selected from
SEQ ID NO: 17 [mFASG113F-TE_WT],
SEQ ID NO: 18 [mFASG113F-'TesA],
SEQ ID NO: 21 [mFASG113W-TE_WT],
SEQ ID NO: 6 [mFASWT-'TesA],
SEQ ID NO: 8 [mFASWT-'TesA4x],
SEQ ID NO: 10 [mFASG113S-'TesA],
SEQ ID NO: 11 [mFASG113S-'TesAL109P],
SEQ ID NO: 12 [mFASG113S-'TesA4x],
SEQ ID NO: 14 [mFASG113M-'TesA],
SEQ ID NO: 15 [mFASG113M-'TesAL109P],
SEQ ID NO: 16 [mFASG113M-'TesA4x],
SEQ ID NO: 19 [mFASG113F-'TesAL109P],
SEQ ID NO: 31 [mFASG113T-'TesA],
SEQ ID NO: 32 [mFASG113L-'TesA],
SEQ ID NO: 33 [mFASG113H-'TesA], and
SEQ ID NO: 34 [mFASG113Y-'TesA];
wherein said method is a method for producing a C₁₄ fatty acid, wherein said protein comprises an amino acid sequence selected from
SEQ ID NO: 9 [mFASG113S-TE_WT],
SEQ ID NO: 13 [mFASG113M-TE_WT],
SEQ ID NO: 17 [mFASG113F-TE_WT],
SEQ ID NO: 21 [mFASG113W-TE_WT],
SEQ ID NO: 6 [mFASWT-'TesA],
SEQ ID NO: 8 [mFASWT-'TesA4x],
SEQ ID NO: 10 [mFASG113S-'TesA],
SEQ ID NO: 11 [mFASG113S-'TesAL109P],
SEQ ID NO: 12 [mFASG113S-'TesA4x],
SEQ ID NO: 14 [mFASG113M-'TesA],
SEQ ID NO: 15 [mFASG113M-'TesAL109P],
SEQ ID NO: 16 [mFASG113M-'TesA4x],
SEQ ID NO: 19 [mFASG113F-'TesAL109P],
SEQ ID NO: 31 [mFASG113T-'TesA],
SEQ ID NO: 32 [mFASG113L-'TesA], and
SEQ ID NO: 33 [mFASG113H-'TesA];
wherein said method is a method for producing a C₁₆ fatty acid, wherein said protein comprises an amino acid sequence selected from
SEQ ID NO: 9 [mFASG113S-TE_WT],
SEQ ID NO: 13 [mFASG113M-TE_WT],
SEQ ID NO: 17 [mFASG113F-TE_WT],
SEQ ID NO: 21 [mFASG113W-TE_WT],
SEQ ID NO: 6 [mFASWT-'TesA],
SEQ ID NO: 8 [mFASWT-'TesA4x],
SEQ ID NO: 11 [mFASG113S-'TesAL109P], and
SEQ ID NO: 15 [mFASG113M-'TesAL109P];
wherein said method is a method for producing a C₁₈ fatty acid, wherein said protein comprises an amino acid sequence selected from
SEQ ID NO: 9 [mFASG113S-TE_WT],
SEQ ID NO: 13 [mFASG113M-TE_WT],
SEQ ID NO: 17 [mFASG113F-TE_WT],
SEQ ID NO: 21 [mFASG113W-TE_WT],
SEQ ID NO: 7 [mFASWT-'TesAL109P],
SEQ ID NO: 11 [mFASG113S-'TesAL109P],
SEQ ID NO: 12 [mFASG113S-'TesA4x], and
SEQ ID NO: 15 [mFASG113M-'TesAL109P],
preferably an amino acid sequence of SEQ ID NO: 7 [mFASWT-'TesAL109P]; or
wherein said method is a method for producing a C₈, C₁₀, and/or C₁₂ fatty alcohol and/or C₈, C₁₀, and/or C₁₂ fatty aldehyde, wherein said protein comprises an amino acid sequence selected from
SEQ ID NO: 28 [mFASG113W-TR2],
SEQ ID NO: 29 [mFASG113W-TR1], and
SEQ ID NO: 30 [mFASG113W-TR3]; or
wherein said method is a method for producing a C₁₆, C₁₈, and/or C₂₀ aldehyde, wherein said protein comprises an amino acid sequence of SEQ ID NO: 26 [mFAS-TR1];
wherein, preferably, said method is
a method for producing a C₈ fatty acid, wherein said protein comprises an amino acid sequence selected from
SEQ ID NO: 20 [mFASG113F-'TesA4x] and
SEQ ID NO: 24 [mFASG113W-'TesA4x], or
a method for producing a C₁₀ fatty alcohol and/or C₁₀ fatty aldehyde, wherein said protein comprises an amino acid sequence selected from
SEQ ID NO: 28 [mFASG113W-TR2],
SEQ ID NO: 29 [mFASG113W-TR1], and
SEQ ID NO: 30 [mFASG113W-TR3].

15. Use of a protein according to any one of claims 1-10, a nucleic acid molecule according to claim 11, or a host cell according to claim 12, for producing a fatty acid, a fatty alcohol, and/or a fatty aldehyde.
